# EUROPEAN PATENT APPLICATION

(11) **EP 4 706 626 A1**
(43) Date of publication of application: **11.03.2026**
(21) Application number: 25199453.9
(22) Date of filing: 01.09.2025
(51) Int. Cl.: A61H 23/02, A61H 39/00

(54) **DATA CABLE AND MASSAGE CONTROL METHOD THEREOF**

(30) Priority: 02.09.2024 CN 202411222848; 30.08.2024 CN 202411213391; 30.08.2024 CN 202422134280 U; 02.09.2024 CN 202422151773 U
(71) Applicant: Dongguan Aikede Electronic Technology Co., Ltd., Dongguan Guangdong (CN)
(72) Inventor: ZHANG, Dehui, Dongguan (CN)
(74) Representative: Metida

(57) **Abstract**

The present invention relates to the field of charging technology, which particularly relates to a data cable and massage control method thereof. The data cable comprises a first input/output module, a first transmission line, a massage module, a second transmission line, and a second input/output module, which are connected in sequence, as well as a main control module electrically connected to the massage module. The first input/output module and the second input/output module are connected to external devices for inputting and outputting electrical signals and data signals. The main control module sends control signals for controlling the start-stop and/or adjustment modes of the massage module. The massage module comprises massage functional components, which perform massage actions according to the control signals. The data cable of the present invention can not only realize basic functions of a data cable such as data transmission and device charging, but also allow users to enjoy massage functions simultaneously. Users can independently control the start, stop and adjustment modes of the massage module with simple and convenient operation, which improves the personalization and flexibility of massage.

## Description

### TECHNICAL FIELD

The present invention relates to the field of charging technology, which particularly relates to a data cable and massage control method thereof.

### BACKGROUND OF THE INVENTION

With the rapid development of science and technology, while people are enjoying the convenience brought by electronic products, they also face health issues arising from the long-term use of such products. The design of traditional data cables often focuses on efficient and stable data transmission and energy supply, yet overlooks the accompanying "digital health" challenges. Maintaining poor postures for a long time when operating electronic devices-such as looking down at screens or operating devices with stiff wrists-has become a hotbed for health problems including stiff necks, muscle soreness, and even cervical spondylosis and carpal tunnel syndrome. These issues not only undermine our work efficiency and creativity, but also silently erode the quality of life, and even pose the risk of evolving into more serious health problems.

In view of this, the innovation of data cable technology is imminent, and there is an urgent need to integrate health concepts and technological innovations to alleviate or even solve the health troubles encountered by users due to long-term use of electronic products at the source. Although a small number of data cables have incorporated physical therapy functions, they suffer from complex structures, inconvenient operation for users, unsatisfactory massage effects, and very limited application scenarios.

### SUMMARY OF THE INVENTION

In order to solve the technical problem of users experiencing health troubles due to prolonged use of electronic products, the present invention provides a data cable and a massage control method thereof.

To solve the above technical problems, the present invention provides the following technical solutions:a data cable, wherein the data cable comprises a first input/output module, a first transmission line, a massage module, a second transmission line, and a second input/output module which are electrically connected in sequence, and a main control module electrically connected to the massage module; the first input/output module and the second input/output module are any one of USB Type-A connector, USB Type-B connector, USB Type-C connector or Lightning connector; the first input/output module is connected to an external device and the second input/output module is connected to another external device for the transmission of electrical or data signals between the two external devices, and simultaneously transmits the electrical signals to the main control module and the massage module; the main control module sends out control signals for controlling the start-stop or adjustment modes of the massage module; the massage module comprises a housing which is bendable, a flexible circuit structure and massage functional components which are disposed within the housing, the data cable comprises a main charging circuit for charging and a massage branch circuit electrically connected to the main charging circuit, the main charging circuit comprises a first transmission line, a flexible circuit structure, and a second transmission line which are electrically connected in sequence; the massage functional components are arranged on the massage branch circuit, positioned along the length direction of the flexible circuit structure, and performs massage actions based on the control signals.

Preferably, the massage module is in the shape of an overall flattened elongated strip; the massage module has a width in the range of 15mm~40mm, a length in the range of 100mm~300mm, and a thickness in the range of 3mm~20mm.

Preferably, the number of the massage functional components is as at least two, and the massage functional components are arranged at intervals in sequence along the length direction of the massage module on the central axis of the massage module; the main control module is also provided with a display device, the display device is an indicator light or a display screen connected to the main control module, which is used for displaying the working status of the massage functional components.

Preferably, the data cable comprises a bidirectional signal recognition circuit for detecting the connection of external devices and recognizing the direction of transmission of electrical and data signals, as long as either the first input/output module or the second input/output module is connected to the external device, the external device can supply power to the main control module and the massage module; the bidirectional signal recognition circuit is implemented by USB OTG technology; the data cable further comprises a first circuit board and a second circuit board, the first transmission line is electrically connected to the flexible circuit structure via the first circuit board, the second transmission line is electrically connected to the flexible circuit structure via the second circuit board.

Preferably, the massage branch circuit further comprises a voltage reduction unit and a voltage boosting unit, which are electrically connected, a first voltage introduced from the first input/output module or a second voltage introduced from the second input/output module is converted into a third voltage via the voltage reduction unit, the third voltage is converted into a fourth voltage via the voltage boosting unit; the third voltage or the fourth voltage is introduced into the massage functional components; the voltage reduction unit and the voltage boosting unit are integrated on the first circuit board or the second circuit board; or the main control module is integrated on the first circuit board or the second circuit board, or the main control module is integrated on a third circuit board independent of the first circuit board and the second circuit board.

Preferably, the main charging circuit comprises at least a positive power transmission line, a ground transmission line, a positive data transmission line, a negative data transmission line, and a configuration channel line; the massage branch circuit leads out at least two transmission pins electrically connected to the massage functional components; the pads of the transmission pins are arranged on the flexible circuit structure, the first circuit board or the second circuit board.

Preferably, the massage functional components are massage electrode sheets, and each massage electrode sheet comprises a main body, as well as conductive pins and a conductive working surface arranged on the main body; the transmission pins comprise an EMS positive pin and an EMS negative pin; the conductive pins of the at least one massage electrode sheet are electrically connected to the EMS positive pin, and the conductive pins of the at least another one massage electrode sheet are electrically connected to the EMS negative pin; or the massage functional components are vibration motors, each vibration motor comprises a motor positive pin and a motor ground pin, the transmission pins comprise a transmission positive pin and a transmission ground pin, the motor positive pins and the motor ground pins are electrically connected to the transmission positive pin and transmission ground pin respectively.

Preferably, the housing is provided with flexible massage parts corresponding to the massage functional components, massage structures are arranged on opposite sides of each massage part, and an elastic structure is arranged on a peripheral side of the massage structure of at least one side of each massage part; each elastic structure comprises a first annular groove, an annular protrusion, and a second annular groove which are connected in sequence; each massage part is provided with a cavity accommodating corresponding massage functional component, and a boss is provided on the inner wall of each cavity.

Preferably, the housing is provided with a matching receiving groove corresponding to the flexible circuit structure; each massage electrode sheet is mounted on the housing via a support member; each support member is provided with a hollow cavity for receiving the main body of corresponding massage electrode sheet, the conductive working surface of each massage electrode sheet is exposed out of the hollow cavity of corresponding support member, a transverse groove communicating with the hollow cavity of each support member is provided on an end of each support member away from the conductive working surface of corresponding massage electrode sheet, and the conductive pins of each massage electrode sheet are exposed out of the transverse groove of corresponding support member; an annular groove is provided on the peripheral side of each support member, and the housing is provided with openings, and each annular groove is clamped with the edge of each opening.

Preferably, the main control module comprises a function control unit, the function control unit being electrically connected to the massage module for receiving user instructions and outputting corresponding control signals to the massage module in accordance with the user instructions; the function control unit comprises a signal input unit, the user instructions being received via the signal input unit; or the user instructions being input via the external device connected to the first input/output module or the external device connected to the second input/output module, and an application program for receiving the user instructions may be provided on the external device; after the user inputting the user instructions by the application program, the user instructions are transmitted to the main control module via the first input/output module or the second input/output module to control the start-stop or adjustment modes of the massage module.

Preferably, the massage actions comprise any one type of, or a combination of any several types of, a vibration signal, an electrical pulse signal, and a heat signal; and the adjustment modes comprise any one type of, or a combination of any several types of, a massage intensity adjustment, a massage mode adjustment, and a timing adjustment.

Preferably, the data cable further comprises a massage accessory which is detachable, the main control module is provided with an output interface, the massage accessory is electrically connected to the main control module via the output interface; the massage accessory comprises any one type of, or a combination of any several types of, a vibration massage member, a heating massage member, an electric pulse massage member.

Preferably, the data cable is further provided with a buckle assembly, the buckle assembly comprises a buckle member and a cable clamp member which are detachably connected, the cable clamp member being provided with a first through-hole, a second through-hole and a locking and unlocking device; both ends of the data cable pass through the first through-hole and the second through-hole respectively and are fixed or movable with respect to the cable clamp member by the locking and unlocking device; or the cable clamp member comprises a male part and a female part which are detachably connected, the first through-hole and the second through-hole are provided in the male part and the female part respectively, and at least one of the male part and the female part is provided with the locking and unlocking device.

To solve the above technical problems, the present invention provides another technical solution as follows:A massage control method for a data cable, the data cable comprises a first input/output module, a massage module and a second input/output module connected in sequence, and a main control module electrically connected to the massage module; the first input/output module is connected to an external device, the second input/output module being connected to another external device, realizing the transmission of electrical or data signals between the two external devices, and simultaneously transmitting electrical or data signals to the main control module and the massage module ; the method comprises:obtaining control signals from the main control module based on user instructions;inputting electrical signals from the first input/output module or the second input/output module to supply power to the massage module; based on the control signals, controlling the massage module to perform massage actions.

Preferably, the method further comprises: controlling different working modes of the data cable based on user instructions; the working modes comprise the data cable transmitting only data signals and/or electrical signals, the data cable transmitting data signals and/or electrical signals while performing massage actions, and the data cable performing only massage actions.

Compared with the prior art, the data cable and massage control method thereof provided by the present invention have the following beneficial effects:
1.The data cable provided by the present invention adds a massage function, enabling the data cable to not only charge the user's electronic devices but also provide massage for the user. Among them, the first input/output module and the second input/output module are used for connecting with external devices; the main charging circuit is used to realize the basic functions of the data cable such as data transmission and device charging. When either one of the first input/output module and the second input/output module is connected to an external electronic device and the other is connected to a power source, the power source can charge the external electronic device through the main charging circuit. The massage branch circuit is used to support the operation of the massage functional components and can obtain the electrical signals required for the operation of the massage module from the main charging circuit, allowing the user to enjoy the massage effect brought by the data cable and relieve physical fatigue. The design of such dual circuits makes the main charging circuit and the massage branch circuit independent of each other without mutual interference. The user can independently control the start-stop and adjustment modes of the massage module through the main control module, realizing three different modes: the data cable only transmits data signals and/or electrical signals, the data cable transmits data signals and/or electrical signals while performing massage actions, and the data cable only performs massage actions. The operation is simple and convenient, which improves the personalization and flexibility of the massage. The first input/output module, the first transmission line, the massage module, the second transmission line and the second input/output module are connected in sequence. Meanwhile, the massage module is provided with a bendable housing and a flexible circuit structure, making the massage module thin and soft, and easy to bend or deform. The massage functional components are arranged along the length direction of the flexible circuit structure. Therefore, the arrangement of the massage module will not add additional branches to destroy the overall linear structure of the data cable, so that the overall appearance of the data cable is not much different from that of traditional data cables, still maintaining an elongated strip shape. The user can use the line structure of the data cable to wind and fix body parts, so that the massage module can fit more closely with the user's massage area, achieving a better massage experience.
2.The data cable provided by the present invention has a massage module that is entirely in the shape of an overall flattened elongated strip. This shape makes the entire data cable lighter and more aesthetically pleasing, and also enhances the bendability of the housing, allowing it to fit more closely to the human skin for massage.The massage module has a width ranging from 15mm to 40mm, a length ranging from 100mm to 300mm, and a thickness ranging from 3mm to 20mm. With such compact dimensions, the massage module will not make the entire data cable bulky due to its addition. It conforms to users' inherent perception of the shape of traditional data cables, improving user acceptance, and better adapting to daily carrying and usage scenarios.
3.The data cable provided by the present invention is equipped with at least two massage functional components. Multiple massage functional components can enhance the massage effect; meanwhile, different massage effects can be achieved through different layouts and cooperation among the multiple massage functional components. The massage functional components are arranged at intervals in sequence along the length direction of the massage module on the central axis of the massage module. Arrangement along the length direction of the massage module can form a wider massage area, achieving multi-dimensional massage effects on the massage part. In addition, there are gaps between the massage functional components arranged at intervals. By changing the size of the gaps, the bending radian of the massage module can be adjusted, making the overall design of the data cable more flexible. The massage functional components are arranged on the central axis of the massage module, which plays a counterweight role, keeping the overall center of gravity of the massage module on its central axis. When the massage module fits the human body for massage, it is not easy to turn up due to the offset of the center of gravity, reducing the twisting and tangling of the data cable during use. A display device is further arranged on the main control module, which facilitates the user to observe the working status of the massage functional components.
4.The data cable provided by the present invention comprises a bidirectional signal identification circuit, which is used to identify the transmission direction of electrical signals and data signals and detect the connection status of external devices. As long as either the first input/output module or the second input/output module is connected to an external device, the external device can supply power to the main control module and the massage module. The bidirectional signal identification circuit is implemented by USB-OTG technology. The first transmission line and the second transmission line are generally circular in shape, and the thickness of their cross-sections is usually relatively large, while the flexible circuit structure is usually thin and soft, and easy to bend or deform. The connection via the first circuit board and the second circuit board can provide mechanical support for the flexible circuit structure, preventing it from being deformed by external forces, thereby ensuring the stability of the connection.
5.The data cable provided by the present invention, the massage branch circuit further comprises a voltage reduction unit and a voltage boosting unit which are electrically connected. The first voltage introduced from the first input/output module or the second voltage introduced from the second input/output module is converted into a third voltage by the voltage reduction unit, and the third voltage is converted into a fourth voltage by the voltage boosting unit. Either the third voltage or the fourth voltage is introduced into the massage functional components. The first voltage and the second voltage can be input from a power source or an external device, such as a mobile phone, a power bank, etc. The first voltage is unstable and has a wide range, and does not support the operation of the massage functional components. The third voltage realizes the standardized processing of the input first voltage and second voltage, which can avoid damage to internal electronic components caused by excessively high or unstable input voltage. Moreover, the voltage boosting unit can facilitate voltage boosting processing more conveniently, converting the third voltage into the fourth voltage required by the massage functional components. This design can adjust the voltage values of the third voltage and the fourth voltage according to requirements to provide flexible power support for the massage functional components, ensuring the stable operation of the massage module. No component will be damaged due to excessively high input voltage, nor will its operating efficiency be reduced due to excessively low voltage. The voltage reduction unit and the voltage boosting unit are integrated on the first circuit board or the second circuit board, which can effectively reduce the number of components and connecting wires, thereby saving space and making the layout of the overall structure more compact. At the same time, it can reduce the wiring length of the circuit, optimize the circuit layout, reduce losses, and improve the overall stability and reliability of the data cable. Arranging the control circuit on the first circuit board or the second circuit board further improves the compactness of the circuit layout, making the overall appearance of the data cable more concise. Alternatively, the main control module is integrated on a third circuit board independent of the first circuit board and the second circuit board, which can reduce mutual interference between the main control circuit and other circuits, improve the maintainability of the product, reduce the difficulty of maintenance, and extend the service life of the device.
6. The data cable provided by the present invention, the main charging circuit comprises at least a positive power transmission line, a ground transmission line, a positive data transmission line, a negative data transmission line, and a configuration channel line. The positive power transmission line and the ground transmission line ensure that the circuit provides stable power supply and good grounding. The positive data transmission line and the negative data transmission line transmit data in different directions respectively, and provide support for the bidirectional transmission of data. The configuration channel line is used to identify the external devices.The massage branch circuit leads out at least two transmission pins to be electrically connected with the massage functional components, so as to realize the normal operation of the massage functional components. Arranging the pads of the transmission pins on the flexible circuit structure can reduce the wiring length when the transmission pins are connected with the massage functional components, facilitating the layout of internal circuits. Arranging the pads of the transmission pins on the first circuit board or the second circuit board can improve the stability of the pads of the transmission pins.
7.The data cable provided by the present invention uses massage electrode sheets as the massage functional components. Each massage electrode sheet comprises a main body, as well as conductive pins and a conductive working surface arranged on the main body. The transmission pins comprise an EMS positive pin and an EMS negative pin. The conductive pins of the at least one massage electrode sheet are electrically connected to the EMS positive pin, and the conductive pins of the at least another one massage electrode sheet are electrically connected to the EMS negative pin. Only when the human body touches the conductive working surfaces of the two massage electrode sheets at the same time, can a complete current loop be formed, and the massage electrode sheets generate an electric shock effect to massage the human body, thus ensuring the safety of the massage. Alternatively, the massage functional components are vibration motors. Each vibration motor comprises a motor positive pin and a motor ground pin, and the transmission pins comprise a transmission positive pin and a transmission ground pin. The motor positive pins and the motor ground pins are electrically connected to the transmission positive pin and the transmission ground pin respectively. This electrical connection method ensures the normal and stable operation of the vibration motors, better adjust the operating states of the vibration motors, and more accurately controls the start, stop and speed of the vibration motor.
8.The data cable provided by the present invention, the housing is provided with flexible massage parts corresponding to the massage functional components , massage structures are arranged on opposite sides of each massage part, which can minimize the attenuation of massage intensity and ensure the massage effect. The flexible massage parts can reduce the pressure of the massage module on the skin, avoid discomfort or pain, and at the same time make the massage force distribution more even, improving the user experience. The massage structures are arranged on opposite sides of each massage part, which provides a richer massage experience. Two different massage effects can be achieved by arranging different massage structures on the two sides. Because an elastic structure is arranged on a peripheral side of the massage structure of at least one side of each massage part, when the massage actions of the massage functional components are vibration signals, the elastic structures increase the movement range of the massage functional components during vibration, expands the vibration space, and at the same time concentrates the vibration effect of the massage functional components on the massage structures, reducing the attenuation caused by the vibration transmitting to other parts of the housing. Each elastic structure comprises a first annular groove, an annular protrusion, and a second annular groove which are connected in sequence, forming an approximately "bow"-shaped structure. When the massage actions of the massage functional components are vibration signals, this design can effectively increase the vibration range of the massage functional components in all directions, achieve a better vibration massage effect, and at the same time effectively prevent the vibration from spreading to other parts. Each massage part is provided with a cavity accommodating corresponding massage functional component , and a boss is provided on the inner wall of each cavity. The bosses can better fix the massage functional components in the cavity. When the massage functional components performs vibration massage, it can fully drive the massage parts to vibrate together, achieving better cooperation with the massage structures and obtaining a better massage experience.
9.The data cable provided by the present invention, is provided with a matching receiving groove on the housing corresponding to the flexible circuit structure. The receiving groove limits and fixes the flexible circuit structure, preventing the flexible circuit structure from twisting and deforming in the housing during use, which may lead to poor electrical contact or open circuit. Each massage electrode sheet is mounted on the housing via a support member. The structure of massage electrode sheets are simple, and the arrangement of the support members facilitate the installation of the massage electrode sheets on the housing, preventing the massage electrode sheets from falling off and being damaged. Each support member is provided with a hollow cavity for receiving the main body of corresponding massage electrode sheet, the conductive working surface of each massage electrode sheet is exposed out of the hollow cavity of corresponding support member , a transverse groove communicating with the hollow cavity of each support member is provided on an end of each support member away from the conductive working surface of corresponding massage electrode sheet, and the conductive pins of each massage electrode sheet are exposed out of the transverse groove of corresponding support member. The hollow cavities enable the massage electrode sheets to be stably installed inside the support members, avoiding loosening or displacement of the massage electrode sheets, thereby ensuring their stable operation during use. The conductive working surface of each massage electrode sheet is directly exposed out of the hollow cavity of corresponding support member, facilitating contact with human skin and forming a good current loop. The transverse groove of each support member allows the conductive pins of corresponding massage electrode sheet to be exposed, facilitating the electrical connection between the conductive pins and the transmission pins. Meanwhile, the transverse groove of each support member allows the flexible circuit structure to pass through below corresponding support member, making the internal layout more compact. An annular groove is provided on the peripheral side of each support member, and the housing is provided with openings, and each annular groove is clamped with the edge of each openings. The clamping connection between each annular groove and each opening can effectively connect the support members and the housing tightly. Moreover, the clamping design simplifies the installation process and reduces the difficulty of production and maintenance.
10.The data cable provided by the present invention, the main control module comprises a function control unit, the function control unit being electrically connected to the massage module for receiving user instructions and outputting corresponding control signals to the massage module in accordance with the user instructions. The user can input different user instructions. The function control unit processes the input user instructions and outputs corresponding control signals to the massage module to control the start-stop and adjustment modes of the massage module. This design simplifies the operation process, allowing the user to control the massage module directly by inputting user instructions, which is convenient and intuitive to use. The function control unit comprises a signal input unit. User instructions are received through the signal input unit. The signal input unit can improve the response speed and accuracy of the function control unit. The signal input unit can be designed for multi-source input, not limited to physical buttons, but also including input methods such as voice recognition, which can meet the needs and preferences of different users. Alternatively, the user instructions being input via the external device connected to the first input/output module or the external device connected to the second input/output module, and an application program for receiving the user instructions may be provided on the external device; after the user inputting the user instructions by the application program, the user instructions are transmitted to the main control module via the first input/output module or the second input/output module to control the start-stop or adjustment modes of the massage module. This design can provide a more intuitive and convenient operation interface on the external device, allowing users to make personalized customizations according to their own preferences.
11. The data cable provided by the present invention has massage actions, which comprise any one type of, or a combination of any several types of, vibration signals, electrical pulse signals and thermal signals. Multiple massage actions can provide a richer massage experience: vibration signals promote muscle relaxation through mechanical action, relieve muscle tension, and effectively alleviate muscle fatigue and soreness; electrical pulse signals can stimulate nerves and muscles to achieve the effects of relieving muscle fatigue and promoting blood circulation; thermal signals transfer heat to the skin and tissues to achieve a hot compress effect. The adjustment modes comprise any one type of, or a combination of any several types of, a massage intensity adjustment, a massage mode adjustment and a timing adjustment. Different adjustment modes can meet the different needs of users: massage intensity adjustment allows users to select the massage intensity according to personal preferences and tolerance; massage mode adjustments can simulate different massage techniques and effects. For example, when the massage action of the massage functional component is a vibration signal, the vibration effect can be from weak to strong, alternately weak and strong, or the vibration rhythm can be alternately fast and slow. Timing adjustment facilitates users to control the massage time and prevent over-massage.
12.The data cable provided by the present invention further comprises a massage accessory which is detachable. The main control module is provided with an output interface, the massage accessory is electrically connected to the main control module via the output interface. The massage accessory is connected and supplied with power through the output interface, allowing the user to use the massage accessory while using the massage module for massage. The massage accessory is detachable, only connected via the output interface when in use, which does not affect the overall appearance and structure of the data cable. Meanwhile, this design also makes the massage accessory easier to maintain and replace. The massage accessory comprises any one type of, or a combination of any several types of, a vibration massage member, a heating massage member, and an electrical pulse massage member. Different massage accessories can achieve different massage effects, and users can choose according to their preferences.
13.The data cable provided by the present invention is further provided with a buckle assembly, the buckle assembly comprises a buckle member and a cable clamp member which are detachably connected, the cable clamp member being provided with a first through-hole, a second through-hole and a locking and unlocking device ; both ends of the data cable pass through the first through-hole and the second through-hole respectively and are fixed or movable with respect to the cable clamp member by the locking and unlocking device. The buckle assembly improves the portability of the data cable. The first transmission line and the second transmission line of the data cable are connected through the cable clamp member, enabling the data cable to form a loop. The user can loop the data cable around parts such as the wrist or ankle, and then adjust the size of the loop through the locking and unlocking device to make it fit perfectly with the body part, thereby achieving a better massage effect. Alternatively, the cable clamp member comprises a male part and a female part which are detachably connected, the first through-hole and the second through-hole are provided in the male part and the female part respectively, and at least one of the male part and the female part is provided with the locking and unlocking device. This design allows the first transmission line and the second transmission line to be easily fastened or unfastened, forming a loop or expanding linearly to meet different usage needs of users.
14.The present invention also provides a data cable massage control method, which is applied to the data cable as described above. The method comprises the following steps: obtaining control signals from the main control module based on user instructions; inputting electrical signals from the first input/output module or the second input/output module to supply power to the massage module; based on the control signals, controlling the massage module to perform massage actions. This method is simple and intuitive to operate. The user only needs to input user instructions to operate the massage module to perform massage actions. The massage module can be powered by either the first input/output module or the second input/output module, which is convenient for the user to operate without distinguishing the direction of the data cable.
15.The massage control method for the data cable provided by the present invention further comprises: controlling different working modes of the data cable based on user instructions; the working modes comprise the data cable transmitting only data signals and/or electrical signals, the data cable transmitting data signals and/or electrical signals while performing massage actions, and the data cable performing only massage actions. The data cable has three different working modes, which can meet the different usage needs of users. This massage control method for the data cable also has the same beneficial effects as the aforementioned data cable, which will not be repeated here.

### BRIEF DESCRIPTION OF DRAWINGS

To more clearly illustrate the technical solutions in the embodiments of the present invention, the accompanying drawings required for describing the embodiments or the prior art will be briefly introduced below. Obviously, the accompanying drawings in the following description are merely some embodiments of the present invention, and those of ordinary skill in the art can also obtain other accompanying drawings based on these drawings without exerting creative efforts.
Fig. 1 is a first schematic framework diagram of the data cable provided by the first embodiment of the present invention.
Fig. 2 is a second schematic framework diagram of the data cable provided by the first embodiment of the present invention.
Fig. 3 is a three-dimensional schematic diagram of the data cable provided by the first embodiment of the present invention.
Fig. 4 is an exploded schematic diagram of the massage module of the data cable provided by the first embodiment of the present invention.
Fig. 5 is a schematic framework diagram of the main control module of the data cable provided by the first embodiment of the present invention.
Fig. 6 is a schematic framework diagram of the function control unit of the data cable provided by the first embodiment of the present invention.
Fig. 7 is a schematic framework diagram of the data cable provided by the second embodiment of the present invention
Fig. 8 is a schematic framework diagram of the main charging circuit of the data cable provided by the second embodiment of the present invention.
Fig. 9 is an exploded schematic diagram of the data cable provided by the second embodiment of the present invention.
Fig. 10 is a schematic framework diagram of the massage branch circuit of the data cable provided by the second embodiment of the present invention.
Fig. 11 is a three-dimensional schematic diagram of the data cable provided by the second embodiment of the present invention.
Fig. 12 is a first schematic circuit connection diagram of the data cable provided by the second embodiment of the present invention.
Fig. 13 is a three-dimensional schematic diagram of the massage electrode sheet of the data cable provided by the second embodiment of the present invention.
Fig. 14 is a second schematic circuit connection diagram of the data cable provided by the second embodiment of the present invention.
Fig. 15 is a three-dimensional schematic diagram of partial components of the data cable provided by the second embodiment of the present invention.
Fig. 16 is a third schematic circuit connection diagram of the data cable provided by the second embodiment of the present invention.
Fig. 17 is a schematic circuit connection diagram of the voltage reduction unit of the data cable provided by the second embodiment of the present invention.
Fig. 18 is a schematic circuit connection diagram of the voltage boosting unit of the data cable provided by the second embodiment of the present invention.
Fig. 19 is an exploded schematic diagram of the massage module of the data cable provided by the third embodiment of the present invention.
Fig. 20 is an exploded schematic diagram of the housing of the data cable provided by the third embodiment of the present invention.
Fig. 21 is a cross-sectional schematic diagram of the massage module of the data cable provided by the third embodiment of the present invention.
Fig. 22 is an enlarged view of Part A in Fig. 21.
Fig. 23 is a three-dimensional schematic diagram of the support member of the data cable provided by the fourth embodiment of the present invention.
Fig. 24 is a cross-sectional schematic diagram of partial components of the data cable provided by the fourth embodiment of the present invention.
Fig. 25 is a three-dimensional schematic diagram of the data cable provided by the fifth embodiment of the present invention.
Fig. 26 is a three-dimensional schematic diagram of the data cable provided by the sixth embodiment of the present invention
Fig. 27 is a first exploded schematic diagram of the buckle assembly of the data cable provided by the sixth embodiment of the present invention.
Fig. 28 is a second exploded schematic diagram of the buckle assembly of the data cable provided by the sixth embodiment of the present invention.
Fig. 29 is a step flow chart of the massage control method for the data cable provided by the seventh embodiment.
Fig. 30 is a step flow chart of Step S1 of the massage control method for the data cable provided by the seventh embodiment.

### Description of Drawing Symbols:

1.Data Cable; 2. Data Cable; 3. Data Cable; 4. Data Cable; 5. Data Cable; 6. Data Cable;
11.First Input/Output Module; 12. Main Control Module; 13. Massage Module; 14. Second Input/Output Module; 15. Data Cable Body; 21. Main Charging Circuit; 22. Massage Branch Circuit; 23. First Circuit Board; 24. Second Circuit Board; 26. Massage Electrode Sheet; 27. Vibration Motor; 30. Massage Part; 31. Elastic Structure; 40. Support Member; 41. Opening; 42. Accommodating Groove; 50. Massage Accessory; 51. Output Interface; 60. Buckle Assembly; 61. Buckle Member; 62. Cable Clamp Member;
   121.Display Device; 122. Function Control Unit; 123. Signal Input Unit; 131. Massage Functional Component; 132. Flexible Circuit Structure; 133. Housing; 210. First Transmission Line; 211. Second Transmission Line; 220. Voltage Reduction Unit; 221. Voltage Boosting Unit; 222. Transmission Pin; 260. Main Body; 261. Conductive Working Surface; 262. Conductive Pin; 300. Massage Structure; 301. Cavity; 302. Boss; 310. First Annular Groove; 311. Annular Protrusion; 312. Second Annular Groove; 400. Hollow Cavity; 401. Transverse Groove; 402. Annular Groove; 620. First Through Hole; 621. Second Through Hole; 622. Locking and Unlocking Device; 623. Placement Groove; 624. Pressing Member; 625. Elastic Member; 626. Groove; 627. Male Part; 628. Female Part.

### DETAILED DESCRIPTION OF THE INVENTION

To make the objectives, technical solutions and advantages of the present invention clearer, the present invention will be further described in detail below with reference to the accompanying drawings and embodiments. It should be understood that the specific embodiments described herein are only used to explain the present invention, and are not intended to limit the present invention.

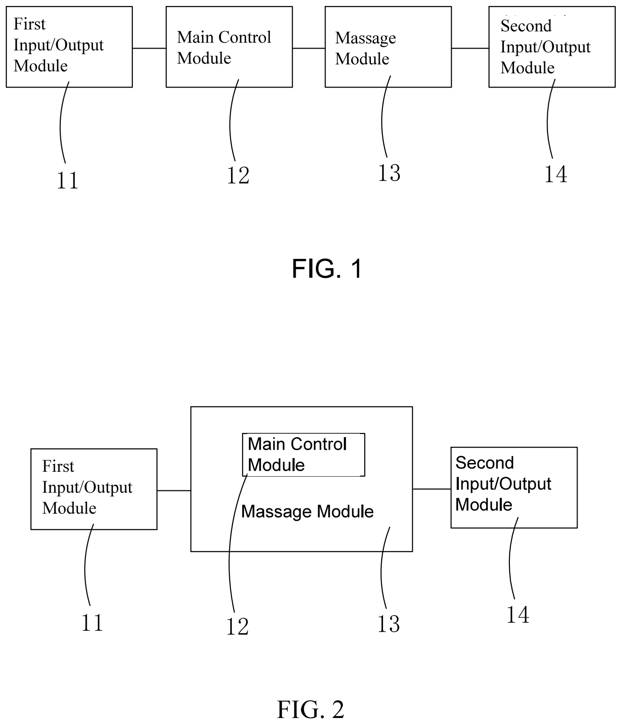

Please refer to Fig. 1. A first embodiment of the present invention provides a data cable 1, which comprises a first input/output module 11, a massage module 13, and a second input/output module 14 electrically connected in sequence, as well as a main control module 12 electrically connected to the massage module 13.

The first input/output module 11 is connected to an external device, and the second input/output module 14 is connected to another external device. They are used for inputting and outputting electrical signals or data signals to realize the transmission of electrical signals or data signals between the two external devices. Meanwhile, they transmit electrical signals to the main control module 12 and the massage module 13 to support the operation of the massage module 13.

It can be understood that the first input/output module 11 and the second input/output module 14 are connected to external electronic devices such as mobile phones and laptops. The first input/output module 11 and the second input/output module 14 realize the functions of data transmission and electrical transmission. When either the first input/output module 11 or the second input/output module 14 is connected to an external device, the external device can supply power to the main control module 12 and the massage module 13.

It can be understood that the first input/output module 11 and the second input/output module 14 can be provided simultaneously, or only one of them can be provided. When only one is provided, the data cable 1 is of single-ended input, and the first input/output module 11 or the second input/output module 14 is only used for supplying power and transmitting data to the main control module 12 and the massage module 13.

Optionally, the first input/output module 11 and the second input/output module 14 can be the same or different. Specifically, the first input/output module 11 and the second input/output module 14 can be USB Type-A connectors, USB Type-B connectors, USB Type-C connectors, Lightning connectors, etc.

The main control module 12 sends control signals to control the start-stop, and/or adjustment modes of the massage module 13.

Further combining Fig. 1 and Fig. 2, the main control module 12 can be provided independently and arranged in a linear layout with the first input/output module 11, the massage module 13, and the second input/output module 14. In this case, the main control module 12 plays a transmission role on the one hand to ensure the normal transmission of electrical signals and data signals of the data cable 1; on the other hand, it serves as a control center to control the massage module 13 start-stop and different adjustment modes of the massage module 13. Users can change the adjustment modes according to their preferences, improving the personalization and flexibility of massage. The main control module 12 can also be integrated with the massage module 13. In this case, the main control module 12 can only serve as a control center without the need to play a transmission role. For the convenience of description, the description of this embodiment takes the independent arrangement of the main control module 12 as an example. It should be noted that the technical solution in which the main control module 12 is integrated into the massage module 13 also falls within the protection scope of this embodiment.

The massage module 13 receives and transmits electrical signals and data signals, and simultaneously receives control signals. The massage module 13 comprises a housing 133 which is bendable, a flexible circuit structure 132 disposed in the bendable housing 133, and massage functional components 131. The massage functional components 131 are arranged along the length extension direction of the flexible circuit structure 132, and perform massage actions according to the control signals.

It can be understood that the massage module 13 plays a transmission role on the one hand to ensure the normal transmission of electrical signals and data signals of the data cable 1. On the other hand, the massage functional components 131 provide massage actions to users according to the control signals to meet their massage needs, so that the data cable 1 can meet the user's massage needs while transmitting electrical signals or data signals.

It can be understood that the first input/output module 11, the main control module 12, the massage module 13, and the second input/output module 14 are connected in sequence, making the overall appearance of the data cable 1 simple and neat, which is convenient for storage and organization. The bendable housing 133 and the flexible circuit structure 132 allow the massage module 13 to be thin and soft, easily bent or deformed. Meanwhile, the massage functional components 131 are arranged along the length direction of the flexible circuit structure 132. Therefore, the arrangement of the massage module 13 does not add additional branches to destroy the overall linear structure of the data cable 1, so that the overall appearance of the data cable 1 is not much different from that of traditional data cables, still maintaining an elongated strip shape. Users can use the wire structure of the data cable 1 to wrap and fix body parts. For example, it can be wrapped and fixed on parts with curves such as wrists and necks, so that the massage module 13 is fixed more stably and fits the human body better, which is convenient for users to operate, increases the application scenarios of the data cable 1 for massage, and achieves better massage experience and effect.

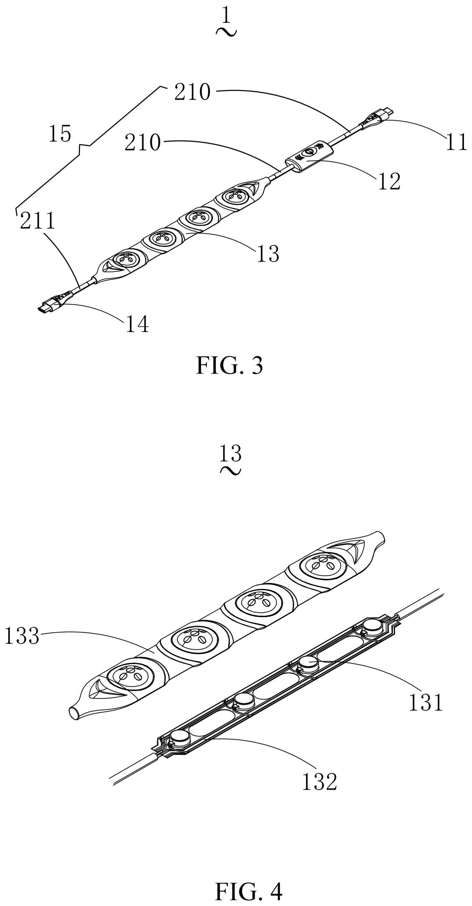

Please further combine Fig. 3. Furthermore, the data cable 1 further comprises a data cable body 15. The data cable body 15 comprises a first transmission line 210, a second transmission line 211, and other transmission lines connecting various modules. The first input/output module 11, the main control module 12, the massage module 13, and the second input/output module 14 are sequentially connected in series through the data cable body 15 to form a continuous bidirectional communication link.

As a specific implementation manner, the first input/output module 11 and the massage module 13 are electrically connected through the first transmission line 210, and the second input/output module 14 and the massage module 13 are electrically connected through the second transmission line 211. The main control module 12 is electrically connected to the massage module 13, and the connection method can be serial connection through the first transmission line 210 or integration with the massage module 13.

It can be understood that the data cable body 15 has the same structure as the wire part of traditional data cables, and can be made of lightweight, soft, and high-performance materials, which is convenient for storage and carrying, and also convenient for users to use the data cable body 15 to wrap and fix the massage module 13. For example, the data cable body 15 can be a nylon braided data cable or a silicone data cable.

Optionally, the diameter of the data cable body 15 ranges from 2mm to 10mm, the width of the massage module 13 ranges from 15mm to 60mm, the length of the massage module 13 ranges from 40mm to 300mm, and the thickness ranges from 3mm to 20mm; the ratio of the width of the massage module 13 to the diameter of the data cable body 15 ranges from 2 to 15.

Preferably, the width of the massage module ranges from 15mm to 40mm, the length ranges from 100mm to 300mm, and the thickness ranges from 3mm to 15mm;

More preferably, the diameter of the data cable body 15 is 5mm, the width of the massage module 13 is 30mm, the length of the massage module 13 is 220mm, and the thickness of the massage module 13 ranges from 3mm to 15mm.

It can be understood that the massage module 13 of this size is compact. Adding the massage module 13 will not make the overall data cable 1 bulky, which conforms to users' inherent perception of the shape of traditional data cables, improves acceptance of users, and better adapts to daily carrying and usage scenarios.

Further, the first input/output module 11, the main control module 12, the massage module 13, the second input/output module 14, and the data cable body 15 of the data cable 1 support the same fast charging protocol, such as the USB-PD (USB Power Delivery) protocol. When the data cable 1 charges an external device, it can increase the charging power and shorten the charging time. At the same time, it can also meet the different voltage and current requirements of the main control module 12 and the massage module 13.

Please further refer to Fig. 4. The massage module 13 further comprises a housing 133 which is bendable and a flexible circuit structure 132 disposed inside the housing 133. The overall structure of the massage module 13 is a flat elongated strip.

It can be understood that to achieve better fit between the massage module 13 and the human body, the massage module 13 adopts the bendable housing 133 to protect the flexible circuit structure 132 and the massage functional components 131. The bendable housing 133 can reduce the impact of collision, wear, etc. on the internal circuits and components of the massage module 13, while increasing the flexibility of the massage module 13 when bent, thus extending its service life. The flexible circuit structure 132 is arranged in an elongated strip shape so as not to damage the linear structure of the data cable 1. The flexible circuit structure 132, i.e., the circuit structure of the massage module 13, has flexibility, which further makes the entire massage module 13 flexible. Combined with the overall wrappable property of the data cable 1, it is convenient for the massage module 13 to better fit the curves of the human body and make closer contact with the massage area, providing users with a better massage experience.

Optionally, the bendable housing 133 can be made of materials such as rubber, plastic, and silicone to achieve its bendable property.

Further, the flexible circuit structure 132 is usually thin and soft, and the massage functional components 131 are arranged at intervals along the extending direction of the flexible circuit structure 132, making the overall thickness of the massage module 13 thin and forming a flat elongated shape similar to a pod. The massage module 13 of this shape is lightweight and aesthetically pleasing, and can better fit the human skin for massage.

Further, at least two massage functional components 131 are provided.

It can be understood that providing multiple massage functional components 131 in the massage module 13 can not only enhance the massage effect. The cooperation between multiple massage functional components 131 can also form a variety of different massage modes, increase the types of adjustment modes controlled by the main control module 12 and bring users a variety of different massage experiences.

Further, the massage functional components 131 are arranged along the length direction of the massage module 13 on the central axis of the massage module 13.

It can be understood that the massage functional components 131 serves as a counterweight. Arranging the massage functional components 131 on the central axis of the massage module 13 can make the overall center of gravity of the massage module 13 lie on the central axis. When the massage module 13 is attached to the human body for massage, it is not easy to turn over due to the offset of the center of gravity, which reduces the twisting and tangling of the data cable 1 during use and improves the user experience.

Further, the wires and components of the flexible circuit structure 132 can be symmetrically arranged on both sides of the central axis of the massage module 13 to play a counterweight role, further ensuring that the overall center of gravity of the massage module 13 is on the central axis. At the same time, the symmetrical arrangement can further improve the performance and stability of the circuit, and help quickly identify each component and connection relationship, thereby improving the readability and maintainability of the flexible circuit structure 132.

Further, the massage functional components 131 are arranged at intervals in sequence along the length direction of the massage module 13.

It can be understood that the massage functional components 131 are arranged along the length direction of the massage module 13, that is, the arrangement direction of the massage functional components 131 are the same as the overall length direction of the data cable 1. This can form a wider massage area and achieve multi-dimensional massage effects on the massage area. For example, when the user wraps the massage module 13 around areas such as the neck and shoulders, multiple massage functional components 131 can massage multiple areas or acupuncture points at the same time, thereby achieving multi-dimensional and multi-level massage effects.

In addition, there are gaps between the massage functional components 131 arranged at intervals. By changing the size of the gaps, the bending radian of the massage module 13 can be adjusted, making the overall design of the data cable 1 more flexible.

Further, the data cable 1 comprises a bidirectional signal recognition circuit. The bidirectional signal recognition circuit is used to detect the connection of external devices and determine the transmission direction of electrical signals and data signals, so as to realize the bidirectional transmission of electrical signals and data signals. That is, electrical signals or data signals can be transmitted from the first input/output module 11 to the second input/output module 14, or from the second input/output module 14 to the first input/output module 11. The main control module 12 and the massage module 13 can be powered by either the external device connected to the first input/output module 11 or the external device connected to the second input/output module 14.

Specifically, the bidirectional signal identification circuit is implemented by USB OTG (On-The-Go) technology, that is, the bidirectional signal identification circuit includes a circuit for implementing the OTG function. In this embodiment, there is no limitation on the specific circuit for implementing the OTG function, and those skilled in the art can adopt any applicable circuit design to implement the OTG function according to actual needs. The first input/output module 11 and the second input/output module 14 with USB OTG function can identify whether an external device is a host or a slave. When an external device is detected as a host, the device can output power to the slave; when an external device is detected as a slave, it can receive power from the host. The USB OTG function can also switch the role of the external device as needed, such as switching from a host to a slave or from a slave to a host. This enables the data cable 1 to use an external device (such as a mobile phone, laptop, etc.) to supply power to the main control module 12 and the massage module 13 as long as one end of the first input/output module 11 or the second input/output module 14 is connected to the external device. This greatly expands the application scenarios of the massage function of the data cable 1, allowing users to massage anytime and anywhere, and improving the user experience.

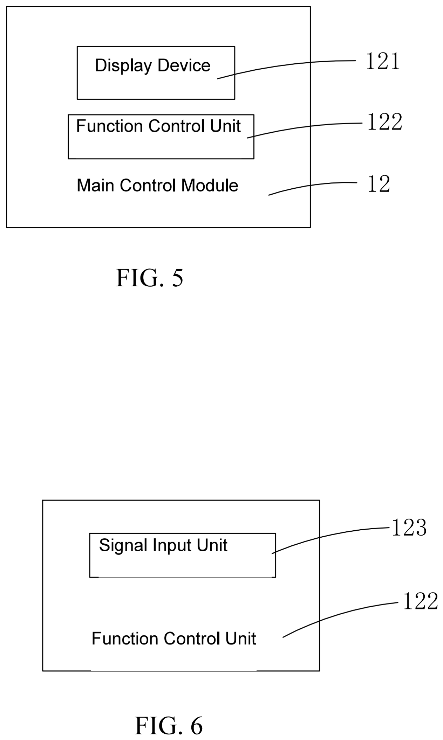

Please further refer to Fig. 5. The main control module 12 comprises a function control unit 122. The function control unit 122 is electrically connected to the massage module 13, and is used to receive user instructions and output corresponding control signals to the massage module 13 according to the user instructions.

It can be understood that the arrangement of the function control unit 122 enables users to control the massage module 13 via the main control module 12. Users can adjust parameters such as the massage mode, intensity, and speed of the massage module 13 according to their own needs and preferences, realizing a personalized massage experience.

It can be understood that the function control unit 122 can further monitor the operating status of the massage module 13. Once an abnormal situation such as overheating or overloading is detected, it can immediately cut off the power supply or adjust the working mode to ensure the safety of users and equipment.

Please further refer to Fig. 6. The function control unit 122 comprises a signal input unit 123, and user instructions are received via the signal input unit 123.

It can be understood that the arrangement of the signal input unit 123 can enhance the interaction capability and flexibility between the user and the massage device. In this embodiment, there is no limitation on the specific form of the signal input unit 123. For example, the signal input unit 123 can be any one type of, or a combination of any several types of a control button, a touch screen, a voice control, a wireless remote control, etc.

Preferably, the signal input unit 123 is control button. There can be one or more control buttons. The control buttons are intuitive and easy to use, have strong adaptability, can reduce misoperations, and have a simple structure without the need for complex circuit design, which can effectively reduce the volume and weight of the massage control system of the data cable 1. In addition, the massage functional components 131 are arranged in parallel on the flexible circuit structure 132, so one-key control by the control buttons can be realized via the control circuit, which simplifies the operation process and allows users to use the massage function of the data cable 1 more easily.

Further, user instructions can also be input via an external device connected to the first input/output module 11 or the second input/output module 14.

It can be understood that an application program for receiving user instructions can be set on the external device. After the user instructions are input by the application program, they are transmitted to the main control module 12 via the first input/output module 11 or the second input/output module 14, thereby controlling the start/stop and adjustment modes of the massage module 13. This design can provide a more intuitive and convenient operation interface on the external device, allowing users to make personalized customizations according to their preferences.

It can be understood that a signal receiver connected to the massage module 13 can be further arranged on the data cable 1 to receive user instructions sent by an external device. That is, the massage module 13 can be controlled even when the external device is not connected to the first input/output module 11 or the second input/output module 14.

Further, the massage actions comprise any one type of, or a combination of any several types of, a vibration signal, an electrical pulse signal, and a heat signal.

It can be understood that the massage actions are performed by the massage functional components 131. By changing the type of the massage functional components 131, the type of the massage action can be changed, and multiple massage actions can provide a richer massage experience. When the massage functional components 131 are vibrators, the massage actions are vibration signals output by the vibrator. The vibration signals promote muscle relaxation through mechanical action, relieves muscle tension, and can effectively alleviate muscle fatigue and soreness. When the massage functional components 131 are pulse generators, the massage actions are electrical pulse signals output by the pulse generator. The electrical pulse signals can stimulate nerves and muscles, achieving effects such as relieving muscle fatigue and promoting blood circulation. When the massage functional components 131 are heaters, the massage actions are thermal signals output by the heater. The thermal signals transfer heat to the skin and tissues, achieving a hot compress effect. The massage actions can also be other types of massage actions output by the massage functional components 131, such as cold compress.

Further, the adjustment modes comprise any one type of, or a combination of any several types of, a massage intensity adjustment, a massage mode adjustment, and a timing adjustment.

It can be understood that different adjustment modes can meet different needs of users. Massage intensity adjustment allows users to select the massage intensity according to personal preferences and tolerance. Massage mode adjustment can simulate different massage techniques and effects. For example, when the massage actions of the massage functional components 131 are vibration signals, the vibration effect can be from weak to strong, alternating between weak and strong, or the vibration rhythm can be alternating between fast and slow. Timing adjustment is convenient for users to control the massage time and prevent over-massage. The types of adjustment modes can be adjusted through the design of the function control circuit of the main control module 12, and each adjustment mode can be further divided into different gears.

Specifically, in this embodiment, the signal input unit 123 of the function control unit 122 is set as one control button. Users can operate this control button to realize the start/stop of the massage module 13 and the adjustment of three gears of massage intensity. Press the control button once to start the massage function of the massage module 13 and adjust the massage intensity of the massage functional component 131 to the first gear; press the control button again to adjust the massage intensity to the second gear; press it again to adjust to the third gear; press it once more to turn off the massage function of the massage module 13. At the same time, long-pressing the control button can also turn off the massage function of the massage module 13.

Further, the data cable 1 can be further provided with an energy storage module. When the first input/output module 11 or the second input/output module 14 is not connected to an external device that provides power, the energy storage module is used to supply power to the main control module 12 and the massage module 13.

Optionally, the energy storage module can be a rechargeable energy storage module or a disposable battery.

It can be understood that when the energy storage module is a rechargeable one, when the first input/output module 11 or the second input/output module 14 is connected to an external device that provides power, the energy storage module can be charged and store energy at the same time, which can be recharged and used multiple times, and has high economy and environmental protection. When the energy storage module is a disposable battery, its structure is simpler, and there is no need to worry about charging and battery life, reducing maintenance costs and time. If the user has no energy storage demand, the disposable battery can be removed to reduce the weight of the data cable 1.

Please continue to refer to Fig. 5. Further, a display device 121 is also electrically connected to the main control module 12.

It can be understood that the display device 121 is convenient for users to observe the working status of the massage functional components 131. Optionally, the display device 121 is an indicator light or a display screen connected to the main control module 12.

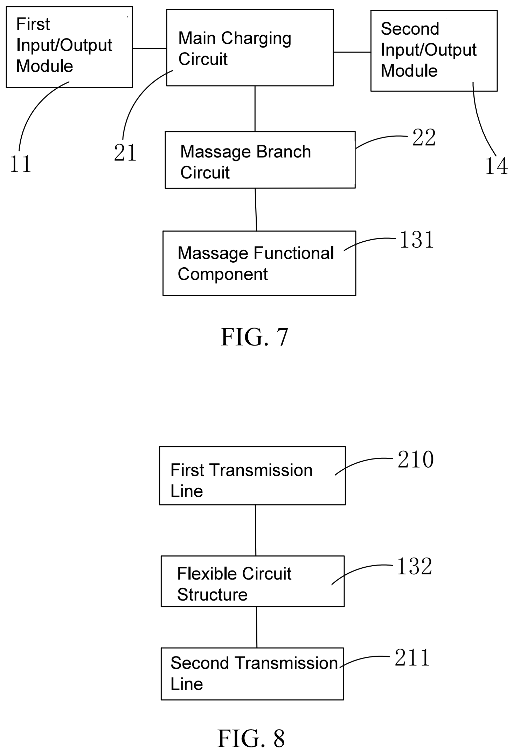

Please refer to Figs. 7 to 9. A second embodiment of the present invention provides another data cable 2. The difference from the above-mentioned data cable 1 is that the data cable 2 includes a main charging circuit 21 for charging, and a massage branch circuit 22 electrically connected to the main charging circuit 21. The main charging circuit 21 includes a first transmission line 210, a flexible circuit structure 132, and a second transmission line 211 electrically connected in sequence. The massage functional components 131 are arranged on the massage branch circuit 22 and along the length direction of the flexible circuit structure 132.

It can be understood that the main charging circuit 21 is used to realize the basic functions of a data cable such as bidirectional power and data transmission, and device charging. When one of the first input/output module 11 and the second input/output module 14 is connected to an external electronic device and the other is connected to a power source, the power source can charge the external electronic device through the main charging circuit 21. The massage branch circuit 22 is used to support and control the operation of the massage functional components 131, and can obtain the electrical signals required for the operation of the massage module 13 from the main charging circuit 21, allowing users to enjoy the massage effect brought by the data cable and relieve physical fatigue.

This dual-circuit design makes the main charging circuit 21 and the massage branch circuit 22 independent of each other without mutual interference. Users can independently control the start, stop and adjustment modes of the massage module 13 through the main control module 12, realizing three different modes: the data cable 1 only transmits data signals and/or electrical signals, the data cable 1 transmits data signals and/or electrical signals while performing massage actions, and the data cable 1 only performs massage actions. The operation is simple and convenient, improving the personalization and flexibility of the data cable's use.

The first transmission line 210, the flexible circuit structure 132, and the second transmission line 211 are electrically connected in sequence, so the overall data cable 2 is still linear. The massage functional components 131 are arranged along the extending direction of the length of the flexible circuit structure 132, without damaging the original linear structure of the data cable 2. This design is convenient for users to wrap the massage functional components 131 around various parts of the body using the data cable 2 to fit closely, so as to receive a better massage effect.

It can be understood that the massage functional components 131 are arranged along the extending direction of the length of the flexible circuit structure 132. This can mean that the massage functional components 131 are only physically connected to the flexible circuit structure 132 and only maintain a linear arrangement with the flexible circuit structure 132 in terms of appearance and structure; it can also mean that part or all of the massage branch circuit 22 is arranged on the flexible circuit structure 132, and the massage functional components 131 are electrically connected to the flexible circuit structure 132.

It can be understood that the first transmission line 210 and the second transmission line 211 form the data cable body 15, and materials that are lightweight, soft, and have good transmission performance should be selected to facilitate storage and carrying. At the same time further facilitate users to wrap the data cable 2 to fit the massage functional components 131 on the massage area. For example, the first transmission line 210 and the second transmission line 211 can be nylon braided data cables or silicone data cables.

Further, the data cable 2 further comprises a first circuit board 23 and a second circuit board 24. The first transmission line 210 is electrically connected to the flexible circuit structure 132 via the first circuit board 23, and the second transmission line 211 is electrically connected to the flexible circuit structure 132 via the second circuit board 24.

In this embodiment, the display device 121 can be arranged on the first circuit board 23 or the second circuit board 24, or can be arranged independently along with the main control module 12.

It can be understood that the cross-sectional thickness of the first transmission line 210 and the second transmission line 211 is usually relatively large, while the flexible circuit structure 132 is usually thin and soft, and is easy to bend or deform. The connection via the first circuit board 23 and the second circuit board 24 can provide mechanical support for the flexible circuit structure 132, preventing it from being deformed by external forces, thereby ensuring the stability of the connection.

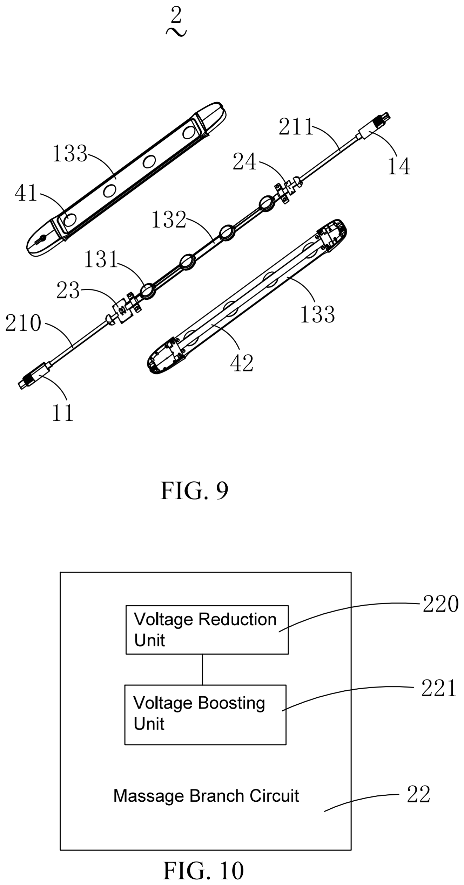

Please further combine Fig. 10. The massage branch circuit 22 comprises a voltage reduction unit 220. The first voltage introduced by the massage branch circuit 22 from the first input/output module 11 or the second voltage introduced from the second input/output module 14 by the massage branch circuit 22 is converted into a third voltage by the voltage reduction unit 220. The first voltage is converted into a unified second voltage by the voltage reduction unit 220, and the second voltage is converted into a third voltage by the voltage boosting unit 221, and the third voltage is introduced into the massage functional components 131.

It can be understood that the first voltage introduced by the massage branch circuit 22 from the first input/output module 11 or the second voltage introduced from the second input/output module 14 actually comes from external devices such as mobile phones and power banks. Since users will connect the data cable 2 to different external devices when using the data cable 2, the first voltage is unstable and has a wide range. The voltage reduction unit 220 converts the first voltage and the second voltage into a unified third voltage, this realizes the standardized processing of the input first voltage, which can avoid damage to internal electronic components caused by excessively high or unstable input voltage.

Further, the massage branch circuit 22 further comprises a voltage boosting unit 221 electrically connected to the voltage reduction unit 220. The third voltage is converted into a fourth voltage by the voltage boosting unit 221, and the fourth voltage is introduced into the massage functional components 131.

It can be understood that the standardized third voltage enables the voltage boosting unit 221 to more conveniently realize the voltage boosting process to convert the third voltage into the fourth voltage required by the massage functional component 131. The voltage boosting unit 221 can adjust the value of the output fourth voltage according to requirements. This design can introduce the third voltage after voltage reduction or the fourth voltage after voltage boosting into the massage functional component 131 according to different types of massage functional components 131, providing flexible power supply support for various types of massage functional components 131.

It can be understood that in this embodiment, there is no limitation on the specific circuit design of the voltage reduction unit 220 and the voltage boosting unit 221. As long as the voltage reduction unit 220 can reduce the input first voltage and second voltage to a unified third voltage, and the voltage boosting unit 221 can boost the third voltage to the fourth voltage required by the massage functional component 131. The third voltage can select different voltage values according to specific needs, such as 5V, 3.3V, etc. The fourth voltage can be set to multiple different voltage value gears corresponding to different massage intensities of the massage functional component 131, such as 24V, 36V, 48V, etc.

Further, the voltage reduction unit 220 and the voltage boosting unit 221 are integrated on the first circuit board 23 or the second circuit board 24.

It can be understood that this design can effectively reduce the number of components and connecting wires, thereby saving space and making the layout of the overall structure more compact. At the same time, it can reduce the wiring length of the circuit, optimize the circuit layout, reduce losses, and improve the overall stability and reliability of the data cable 2.

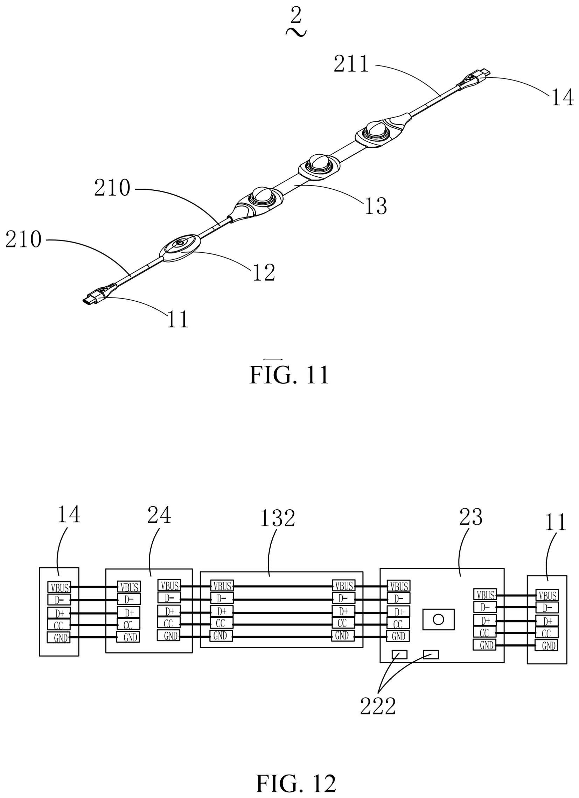

Further, please refer to Figs. 9 and 11. The main control module 12 is integrated on the first circuit board 23 or the second circuit board 24, or the main control module 12 is independently arranged separately from the first circuit board 23 and the second circuit board 24.

It can be understood that arranging the main control module 12 on the first circuit board 23 or the second circuit board 24 facilitates the overall circuit layout, further improves the compactness of the circuit layout, and makes the overall appearance of the data cable 2 more concise.

It can be understood that the main control module 12 can also be arranged independently, for example, integrated on a third circuit board (not shown) independent of the first circuit board 23 and the second circuit board 24. This design can reduce mutual interference between the main control circuit 12 and other circuits, improve the maintainability of the product, reduce the difficulty of maintenance, and extend the service life of the device.

In the embodiment of the present invention, for the convenience of explanation, the voltage reduction unit 220, the voltage boosting unit 221, and the main control module 12 are all integrated on the first circuit board 23 as an example for detailed description. It should be clarified that based on the technical concept of the present invention, the implementation mode in which functional circuits such as the voltage reduction unit 220, the voltage boosting unit 221, and the main control module 12 are integrated on the second circuit board 24 has essentially the same technical principle, implementation logic, and achieved technical effects as the case of integration on the first circuit board 23, and all fall within the protection scope of the present invention.

As a specific embodiment, the main control module 12 is arranged on the first circuit board 23. A control button is provided on the main control module 12. The user can start or stop the operation of the massage functional component 131 through the control button, or adjust the gear of the massage functional component 131, and the display state of the display device 121 will change accordingly. The control button and the display device 121 are arranged on opposite sides of the first circuit board 23.

Please refer to Fig. 12. Further, the main charging circuit 21 comprises at least a positive power transmission line VBUS, a ground transmission line GND, a positive data transmission line D+, a negative data transmission line D-, and a configuration channel line CC.

It can be understood that the positive power transmission line VBUS and the ground transmission line GND ensure that the circuit provides stable power supply and good grounding. The positive data transmission line D+ and the negative data transmission line D-transmit data in different directions respectively, supporting the bidirectional transmission of data through the two positive and negative lines. The configuration channel line CC is used to identify the external device, helping the device identify whether it is a host device or an external device, and determine the communication protocol and power supply method.

Further, the massage branch circuit 22 leads out at least two transmission pins 222 electrically connected to the massage functional components 131, so as to realize the normal operation of the massage functional components 131.

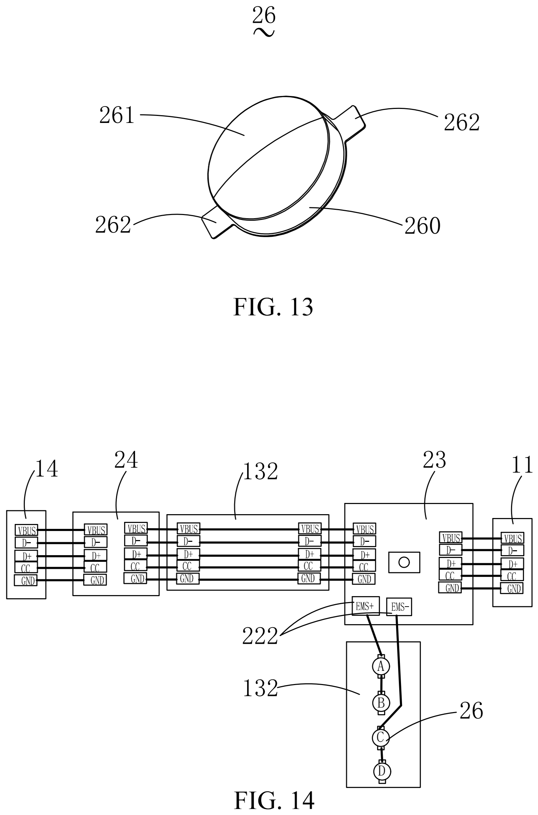

Please refer to Figs. 13 to 15. As a specific embodiment, the massage functional components 131 are massage electrode sheets 26. Each massage electrode sheet 26 comprises a main body 260, as well as conductive pins 262 and a conductive working surface 261 arranged on the main body 260. The transmission pins 222 comprises an EMS (Electrical Muscle Stimulation) positive pin EMS+ and an EMS negative pin EMS-. The conductive pins 262 of the at least one massage electrode sheet 26 are electrically connected to the EMS positive pin EMS+ and the conductive pins 262 of at least another one massage electrode sheet 26 are electrically connected to the EMS negative pin EMS-.

It can be understood that when the human body touches the conductive working surfaces 261 of the two massage electrode sheets 26 at the same time, a path from the EMS positive pin EMS+ to the human body and then to the EMS negative pin EMS- is formed, thereby forming a complete current loop. The massage electrode sheets 26 generate an electric shock effect to massage the human body, ensuring the safety of the massage. In addition, by controlling the intensity of the voltage signal output by the EMS positive pin EMS+ and the EMS negative pin EMS-, the working intensity of the massage electrode sheets 26 can be controlled.

It can be understood that when two massage electrode sheets 26 are provided, one is connected to the EMS positive pin EMS+, and the other is connected to the EMS negative pin EMS-. When multiple massage electrode sheets 26 are provided, they can be connected in series or in parallel, but it is necessary to ensure that at least one massage electrode sheet 26 is connected to the EMS positive pin EMS+, at least another one massage electrode sheet 26 is connected to the EMS negative pin EMS-, and each massage electrode sheet 26 connected to the EMS positive pin EMS+ is not connected to each massage electrode sheet 26 connected to the EMS negative pin EMS-. That is, in the overall design of the data cable 2, the EMS positive pin EMS+ and the EMS negative pin EMS- are not connected; a complete loop can only be formed after contacting the human body.

Specifically, in this embodiment, four massage electrode sheets 26 are provided, namely massage electrode sheet A, massage electrode sheet B, massage electrode sheet C, and massage electrode sheet D. One conductive pin 262 of massage electrode sheet A is connected to the EMS positive pin EMS+, and the other conductive pin 262 is connected in series with massage electrode sheet B. One conductive pin 262 of massage electrode sheet C is connected to the EMS negative pin EMS-, and the other conductive pin 262 is connected in series with massage electrode sheet D. When the human body touches massage electrode sheet A and/or massage electrode sheet B, and at the same time touches massage electrode sheet C and/or massage electrode sheet D, a current loop is formed, and the massage electrode sheets 26 in contact with the human body generate electrical pulses to massage the human body.

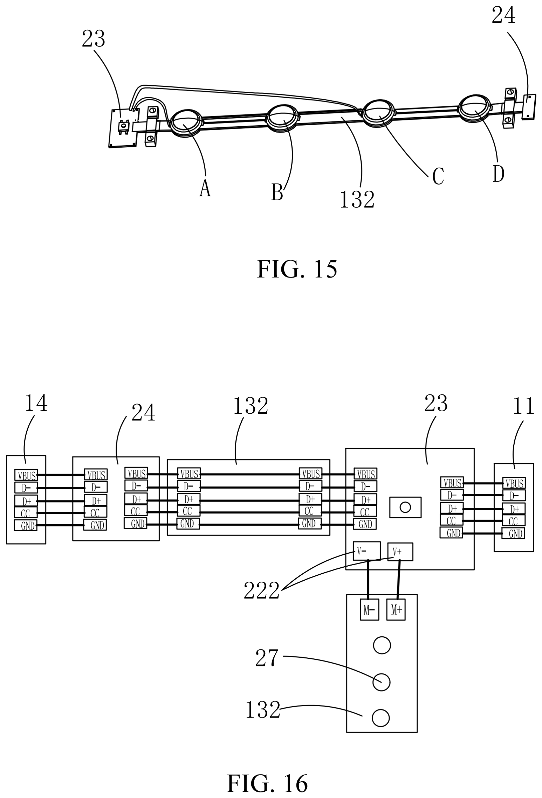

Please refer to Fig. 16. As another specific embodiment, the massage functional components 131 are vibration motors 27. Each vibration motor 27 comprises a motor positive pin M+ and a motor ground pin M-. The transmission pins 222 comprise a transmission positive pin V+ and a transmission ground pin V-. The motor positive pins M+ and the motor ground pins M- are electrically connected to the transmission positive pin V+ and the transmission ground pin V- respectively.

It can be understood that the transmission positive pin V+ and the transmission ground pin V- ensure the normal and stable operation of the vibration motor 27, and can better adjust the operating state of the vibration motor 27, controlling its start, stop, and speed change more accurately.

It can be understood that the two flexible circuit structures 132 in Figs. 14 and 16 can be the front and back sides of the flexible circuit structure 132. The main charging circuit 21 is arranged on the front side of the flexible circuit structure 132, and the massage functional components 131 are arranged on the back side along the extending direction of the flexible circuit structure 132, preventing the massage branch circuit 22 from interfering with the power and data transmission of the main charging circuit 21.

Further, the pads of the transmission pins 222 are arranged on the flexible circuit structure 132, the first circuit board 23, or the second circuit board 24.

It can be understood that arranging the pads of the transmission pins 222 on the flexible circuit structure 132 can reduce the wiring length when the transmission pins 222 are connected to the massage functional components 131, facilitating the layout of internal circuit. Arranging the pads on the first circuit board 23 or the second circuit board 24 can enhance the stability of the pads of the transmission pins 222.

It should be understood that the at least two transmission pins 222 led out by the massage branch circuit 22 refer to two pins in an abstract sense. For example, the motor ground pin M- of the vibration motor 27 needs to be connected to the transmission ground pin V-, and the transmission ground pin V- is actually a ground pin. Therefore, the transmission ground pin V- can be any point on the ground transmission line GND on the first circuit board 23 or the flexible circuit structure 132, that is, the motor ground pin M-can be directly connected to the ground transmission line GND. In this case, the only transmission pin 222 led out by the massage branch circuit 22 is the transmission positive pin V+.

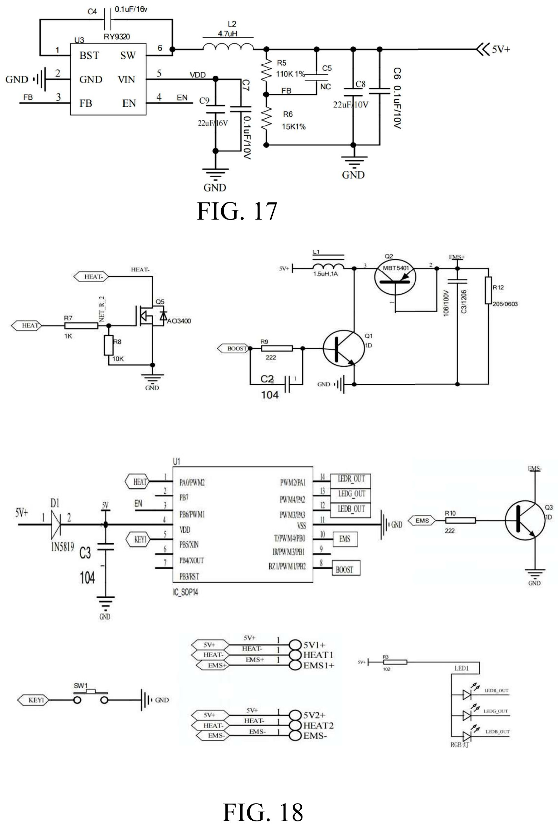

As a specific embodiment, please refer to Figs. 17 and 18. The massage functional components 131 are massage electrode sheets 26. The voltage reduction unit 220 comprises a voltage reduction chip U3 and a circuit arranged around the chip U3. The chip U3 is RY9320, a high-frequency, synchronous, rectified, step-down switching mode converter. The first voltage obtained from the main charging circuit 21 is connected to the pin VIN of the chip U3, and after conversion by the chip RY9320 and its peripheral circuits, a 5V second voltage is output.

The chip U1 and the circuit arranged around U1 constitute the voltage boosting unit 221 and the main control module 12. The chip U1 adopts an IC-SOP14 package. The 5V second voltage is input to the pin VDD of the chip U1, and after processing by the chip U1 and the peripheral circuits, an EMS+ signal and an EMS- signal are output through the pin EMS. These two signals are respectively connected to the EMS positive pin EMS+ and the EMS negative pin EMS-, and then connected to different massage electrode sheets 26. When the human body touches the massage electrode sheet 26 connected to the EMS positive pin EMS+ and the massage electrode sheet 26 connected to the EMS negative pin EMS- at the same time, a complete current loop can be formed to experience electrical pulse massage.

The 5th pin PB5/XIN of the chip U1 is connected to a control switch SW1. Through the control switch SW1, the start, stop, and gear adjustment of the connected massage electrode sheets 26 can be realized.

The 12th, 13th, and 14th pins of the chip U1 are connected in parallel with three RGB lights, which serve as the display device 121. The three RGB lights can indicate that the massage electrode sheets 26 have three adjustable gears. It is convenient for users to observe the working state of the massage functional components 131.

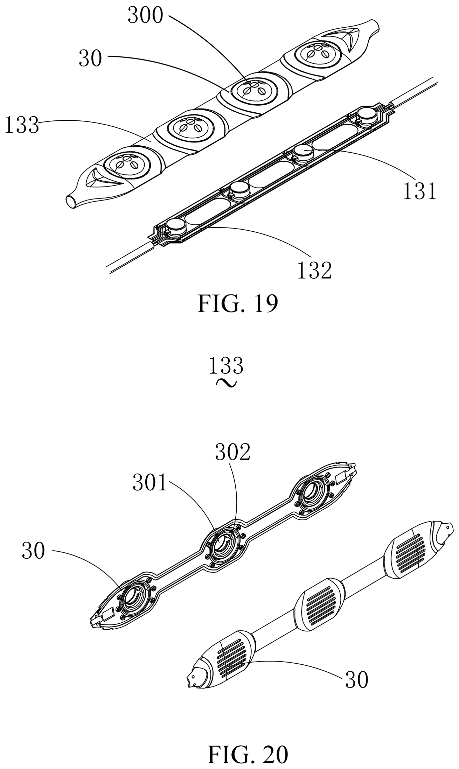

Please refer to Figs. 19 and 20. A third embodiment of the present invention provides another data cable 3. The differences from the above-mentioned data cable 1 or data cable 2 are as follows:

The housing 133 is provided with flexible massage parts 30 corresponding to the massage functional components 131, and massage structures 300 are arranged on opposite sides of each massage part 30.

It can be understood that the flexible massage parts 30 can not only reduce the attenuation of massage intensity, but also reduce the pressure of the massage module 13 on the skin to avoid discomfort or pain. At the same time, it can make the massage force distribution more even, improving the user experience.

It can be understood that arranging massage structures 300 on opposite sides of each massage part 30 can provide a richer massage experience. Optionally, the massage structures 300 on the two sides can be the same or different. Specifically, in this embodiment, there is no limitation on the shape of the massage structures 300. For example, the massage structure 300 can be a convex arc structure, a strip groove, an array of convex points and concave points, a wave structure, a spiral structure, a spherical structure, etc.

Further, each massage part 30 is provided with a cavity 301 for accommodating corresponding massage functional component 131, and a boss 302 is provided on the inner wall of each cavity 301.

It can be understood that the boss 302 can better fix the massage functional component 131 in the cavity 301, preventing the massage functional component 131 from shifting during vibration massage, and effectively driving the massage part 30 to vibrate together, thereby better cooperating with the massage structure 300 to obtain a better massage experience.

It can be understood that when the massage functional components 131 are vibrators, the shape of the massage parts 30 can be set according to the specific shape of the massage functional components 131 to achieve tight wrapping of the massage functional components 131. When the massage functional components 131 vibrate, the tight wrapping can reduce the impact of air damping and other external factors on the vibration, allowing the vibration energy to be more effectively transmitted to the target object, that is, the massage functional components 131 can better drive the massage structures 300 to vibrate, reducing energy loss.

Specifically, in this embodiment, the massage structure 300 on one side of each massage part 30 of the data cable 3 is set as a protrusion with a smooth surface. A cavity 301 matching the massage functional component 131 is opened inside the protrusion. An annular boss 302 is provided on the inner wall of each cavity 301. The annular boss 302 is provided with a notch corresponding to the connecting pin of the massage functional component 131. The main body part of the massage functional component 131 is fixed in the cavity 301 through the annular boss 302, and the connecting pin of the massage functional component 131 is exposed from the notch to connect with the flexible circuit structure 132.

It can be understood that when the massage functional components 131 are vibrators, the part of the housing 133 between the massage parts 30 can be designed to be as thin and light as possible, and its width can be further narrowed. This can reduce vibration attenuation, avoid mutual influence between the massage functional components 131, and at the same time reduce the overall weight of the data cable 3.

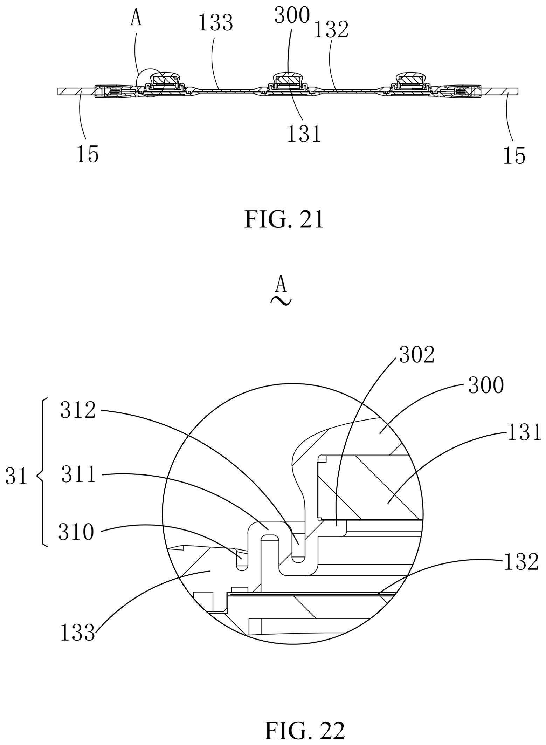

Please further combine Figs. 21 and 22. An elastic structure 31 is provided on a peripheral side of the massage structure 300 of at least one side of each massage part 30 on the data cable 3.

It can be understood that when the massage functional components 131 are vibrators, the elastic structures 31 can increase the movement range of the massage functional components 131 when they vibrates, expand the vibration space, and ensure the massage effect. At the same time, it can concentrate the vibration effect of the massage functional components 131 on the massage structures 300, reducing the attenuation caused by the vibration transmitted to the rest of the housing 133, thereby achieving a better vibration massage experience.

Optionally, each elastic structure 31 comprises a first annular groove 310, an annular protrusion 311, and a second annular groove 312 connected in sequence.

It can be understood that each elastic structure 31 forms an approximately "bow"-shaped structure through the first annular groove 310, the annular protrusion 311, and the second annular groove 312 connected in sequence. This can effectively increase the vibration range of the massage functional components 131 in all directions, obtain a better vibration massage effect, and at the same time effectively prevent the vibration from spreading to other parts.

It can be understood that the rest structure of the data cable 3 is the same as that of the data cable 1 or data cable 2, and has the same beneficial effects, which will not be repeated here.

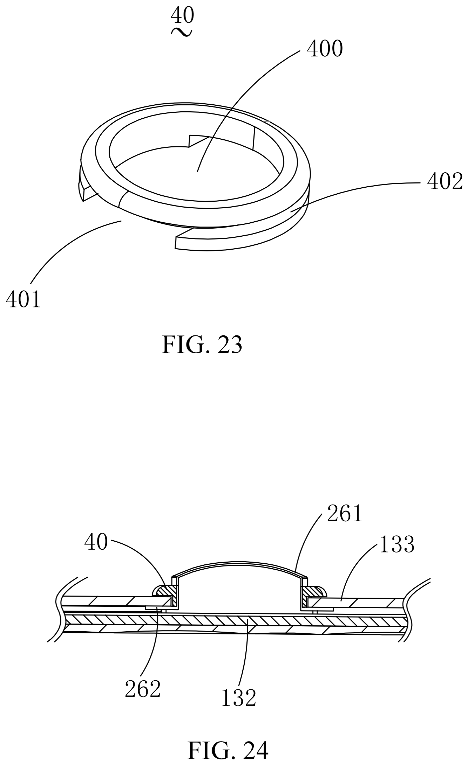

Please further combine Figs. 23 and 24. A fourth embodiment of the present invention provides another data cable 4. The differences from the above-mentioned data cable 1, data cable 2, or data cable 3 is that when each massage functional component 131 is a massage electrode sheet 26, each massage electrode sheet 26 is installed on the housing 133 via a support member 40.

It can be understood that the structures of massage electrode sheets 26 are simple. The arrangement of the support members 40 can more conveniently install the massage electrode sheets 26 on the housing 133, preventing the massage electrode sheets 26 from falling off or being damaged.

Further, each support member 40 is provided with a hollow cavity 400 for accommodating the main body 260 of corresponding massage electrode sheet 26. The conductive working surface 261 of each massage electrode sheet 26 is exposed from the hollow cavity 400 of corresponding support member. A transverse groove 401 communicating with the hollow cavity 400 is opened at an end of each support member 40 away from the conductive working surface 261 of corresponding massage electrode sheet 26, and the conductive pins 262 of each massage electrode sheet 26 are exposed from the transverse groove 401 of corresponding support member 40.

It can be understood that the design of the hollow cavities 400 enable the massage electrode sheets 26 to be stably installed inside the support members 40, avoiding loosening or shifting of the massage electrode sheets 26, thereby ensuring their stable operation during use. The conductive working surface 261 of each massage electrode sheet 26 is directly exposed from the hollow cavity 400 of corresponding support member 40, facilitating contact with human skin and forming a good current loop.

It can be understood that the design of the transverse grooves 401 allow the conductive pins 262 to be exposed, facilitating the electrical connection between the conductive pins 262 and the transmission pins 222. At the same time, the design of the transverse grooves 401 allow the flexible circuit structure 132 to pass below support members 40, making the internal layout more compact.

Further, please combine Figs. 9, 23, and 24. An annular groove 402 is provided on the peripheral side of each support member 40. The housing 133 is provided with openings 41, and each annular groove 402 is clamped with the edge of each opening 41.

It can be understood that the clamping method between the annular grooves 402 and the openings 41 can effectively connect the support members 40 and the housing 133 tightly. The clamping design makes the installation process simpler, reducing the difficulty of production and maintenance.

It should be understood that the support members 40 can also be connected to the housing 133 by other methods besides clamping, such as gluing.

Further, the support members 40 and the massage electrode sheets 26 are integrally formed by insert molding.

It can be understood that this design forms a tight physical integrated connection between the support members 40 and the electrode sheets, avoiding loosening, falling off, or poor contact that may occur in traditional connection methods, and ensuring the stability of the massage electrode sheets 26 during operation. It also eliminates the need for additional connection or fixing steps in the traditional assembly process, simplifying the production process and improving production efficiency.

Further, please continue to refer to Fig. 9. The housing 133 is provided with a matching accommodating groove 42 corresponding to the flexible circuit structure 132.

It can be understood that the accommodating groove 42 limits and fixes the flexible circuit structure 132, preventing the flexible circuit structure 132 from twisting or deforming in the housing 133 during use, which may lead to poor electrical contact or open circuit.

It can be understood that the transverse grooves 401 of the support members 40 should be arranged in the same direction as the accommodating groove 42, facilitating the flexible circuit structure 132 to pass under the support member 40, and reducing the bending and twisting of the wires connecting the conductive pin 262 and the transmission pin 222, thereby improving the reliability of the connection.

Specifically, in this embodiment, the housing 133 comprises a detachable upper housing and lower housing. The openings 41 are provided on the upper housing. Both the upper housing and the lower housing are provided with matching accommodating grooves 42 corresponding to the flexible circuit structure 132.

Further, the housing 133 comprises end caps arranged at both ends for protecting the first circuit board 23 and the second circuit board 24. Each end cap also comprises detachable upper cover and lower cover, and the end caps can be flexible or rigid.

It can be understood that the rest structure of the data cable 4 is the same as that of the data cable 1, data cable 2, or data cable 3, and has the same beneficial effects, which will not be repeated here.

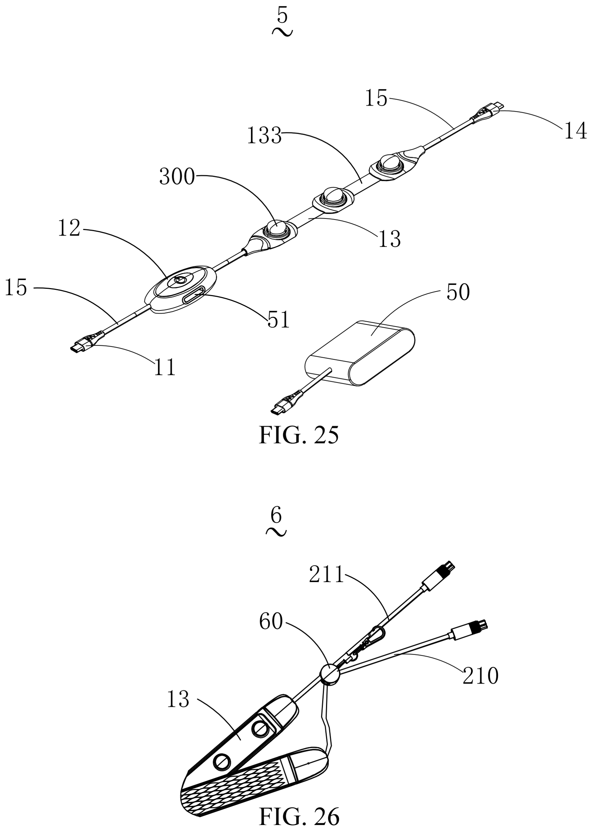

Please refer to Fig. 25. A fifth embodiment of the present invention provides another data cable 5. The difference from the above-mentioned data cable 1, data cable 2, data cable 3, or data cable 4 is as follows:

The data cable 5 further comprises a massage accessory 50 which is detachable. The main control module 12 is provided with an output interface 51, and the massage accessory 50 is electrically connected to the main control module 12 via the output interface 51.

It can be understood that the massage accessory 50 is provided with a connector matching the output interface 51, and power is supplied via the connector and the output interface 51. This design allows the user to use the massage accessory 50 while massaging via the massage module 13. The detachable design of the massage accessory 50 means it is only connected and powered via the output interface 51 when needed, without affecting the overall appearance structure of the data cable 5, and at the same time making the massage accessory 50 easier to maintain and replace.

Further, the massage accessory 50 comprises any one type of, or a combination of any several types of, a vibration massage member, a heating massage member, and an electrical pulse massage member.

It can be understood that different massage accessories 50 can achieve different massage effects. Users can choose according to their preferences, and select the massage accessory 50 to massage other parts while using the massage module 13 for massage.

It can be understood that when multiple different massage components are provided as the massage accessory 50, they can be further arranged in parallel or in series. This design can enhance the flexibility of the massage accessory 50 for massage, provide users with a richer massage experience, and facilitate centralized control, simplifying the user's operation process.

It can be understood that the detachable arrangement of the massage accessory 50 allows for more flexible design of its appearance and style. Specifically, in this embodiment, there is no specific limitation on the shape and structure of the massage accessory 50. Specific massage accessories 50 can be set for body parts prone to fatigue, for example, the massage accessory 50 is a heated eye mask.

It can be understood that the rest structure of the data cable 5 is the same as that of the data cable 1, data cable 2, data cable 3, or data cable 4, and has the same beneficial effects, which will not be repeated here.

Please refer to Fig. 26. A sixth embodiment of the present invention provides another data cable 6. The differences from the above-mentioned data cable 1, data cable 2, data cable 3, data cable 4, or data cable 5 is that a buckle assembly 60 is further provided on the data cable 6.

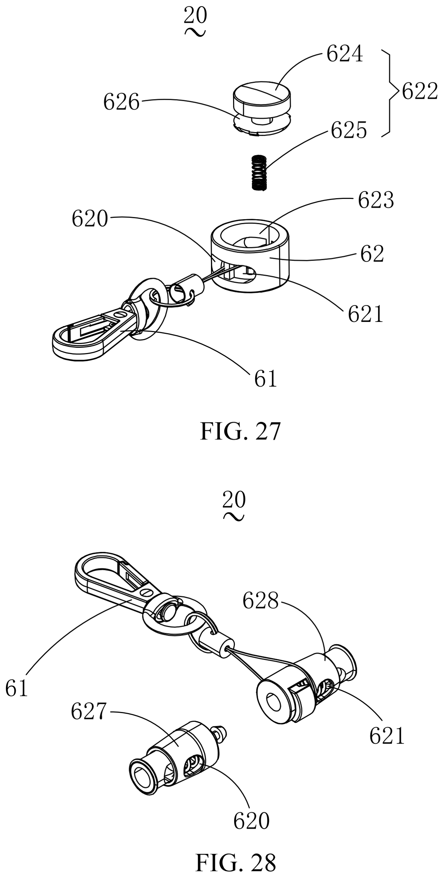

Further, please refer to Fig. 27. The buckle assembly 60 comprises a buckle member 61 and a cable clamp member 62 which are detachably connected. The cable clamp member 62 is provided with a first through hole 620, a second through hole 621, and a locking and unlocking device 622. Both ends of the data cable 6, namely the first transmission line 210 and the second transmission line 211, pass through the first through hole 620 and the second through hole 621 respectively, and are fixed or moved relative to the cable clamp member 62 through the locking and unlocking device 622.

It can be understood that the buckle assembly 60 improves the portability of the data cable 6. The two ends of the data cable 6 are connected through the cable clamp member 62, so that the data cable 6 can form a ring. The user can put the data cable 6 around area such as the wrist, ankle, and neck, and then adjust the size of the ring through the locking and unlocking device 622 to make it fit the body area completely, so as to obtain a better massage effect.

In this embodiment, there is no limitation on the specific structure of the buckle member 61. Optionally, the buckle member 61 can be a D-ring buckle, an O-ring buckle, an figure-eight buckle, etc.

It can be understood that in this embodiment, there is no limitation on the specific structure of the locking and unlocking device 622.

As a specific embodiment, the cable clamp member 62 is provided with a placement groove 623. The placement groove 623 communicates with the first through hole 620 and the second through hole 621. The locking and unlocking device 622 comprises a pressing member 624 and an elastic member 625 abutting against the pressing member 624. The pressing member 624 and the elastic member 625 are placed in the placement groove 623, and the pressing member 624 is provided with a groove 626. The first transmission line 210 and the second transmission line 211 pass through the first through hole 620 and the second through hole 621 and are clamped in the groove 626 at the same time. When the pressing member 624 is pressed down, the elastic member 625 is squeezed by the pressing member 624 and the bottom surface of the placement groove 623, the groove 626 is aligned with the first through hole 620 or the second through hole 621, and the first transmission line 210 and the second transmission line 211 can slide freely relative to the cable clamp member 62. When the pressing member 624 is released, the resilience of the elastic member 625 resets the pressing member 624, and the groove 626 is misaligned with the first through hole 620 or the second through hole 621, so the first transmission line 210 and the second transmission line 211 are locked relative to the cable clamp member 62.

Please further refer to Fig. 28. The cable clamp member 62 comprises a male part 627 and a female part 628 which are detachably connected. The first through hole 620 and the second through hole 621 are provided on the male part 627 and the female part 628 respectively.

It can be understood that the detachable male part 627 and female part 628 allow the first transmission line 210 and the second transmission line 211 to be easily buckled or unbuckled. The entire data cable 6 can be conveniently formed into a ring or unfolded into a linear shape, meeting different usage needs of users.

It can be understood that when the cable clamp member 62 comprises the detachably connected male part 627 and female part 628, at least one of the male part 627 and the female part 628 is provided with the locking and unlocking device 622.

It can be understood that in this embodiment, there is no specific limitation on the detachable connection method between the male part 627 and the female part 628. A protrusion can be provided on the male part 627 and a groove 626 on the female part 628 to realize the detachable connection. Alternatively, magnetic members can be respectively provided on the male part 627 and the female part 628 to realize the detachable connection.

It can be understood that the rest structure of the data cable 6 is the same as that of the data cable 1, data cable 2, data cable 3, data cable 4, or data cable 5, and has the same beneficial effects, which will not be repeated here.

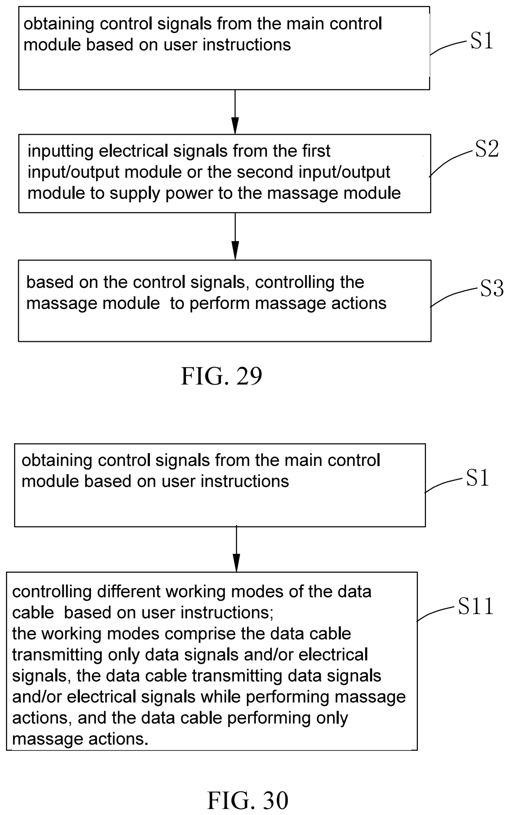

Please refer to Fig. 30. A seventh embodiment of the present invention provides a data cable massage control method. The method is applied to the above-mentioned data cable 1, data cable 2, data cable 3, data cable 4, data cable 5, or data cable 6, and comprises the following steps:
Step S1: Obtaining the control signals of the main control module based on the user instructions;
Step S2: Inputting electrical signals from the first input/output module or the second input/output module to supply power to the massage module;
Step S3: Based on the control signals, control the massage module to perform massage actions .

It can be understood that this method is simple and intuitive to operate. The user only needs to input user instructions to operate the massage module to perform massage actions. The massage module can be powered by either the first input/output module or the second input/output module, which is convenient for the user to operate without distinguishing the direction of the data cable.

Step S1 of the data cable massage control method further comprises the following steps:

Step S11: Controlling different working modes of the data cable based on user instructions; the working modes include the data cable transmitting only data signals and/or electrical signals, the data cable transmitting data signals and/or electrical signals while performing massage actions, and the data cable performing only massage actions.

It can be understood that in this data cable massage control method, the data cable has three different working modes, which can meet different usage needs of users. The user only needs to input the corresponding user instructions to select different working modes, and the operation is simple. This method also has the same beneficial effects as the above-mentioned data cable 1, data cable 2, data cable 3, data cable 4, data cable 5, and data cable 6, which will not be repeated here.

In the embodiments provided by the present invention, it should be understood that "B corresponding to A" means that B is associated with A, and B can be determined according to A. However, it should also be understood that determining B according to A does not mean that B is determined only according to A, and B can also be determined according to A and/or other information.

It should be understood that the reference to "one embodiment" or "an embodiment" throughout the specification means that a specific feature, structure, or characteristic related to the embodiment is included in at least one embodiment of the present invention. Therefore, the appearances of "in one embodiment" or "in an embodiment" in various places throughout the specification do not necessarily refer to the same embodiment. In addition, these specific features, structures, or characteristics can be combined in any suitable manner in one or more embodiments. Those skilled in the art should also know that the embodiments described in the specification are all optional embodiments, and the actions and modules involved are not necessarily required by the present invention.

In various embodiments of the present invention, it should be understood that the sequence numbers of the above processes do not necessarily mean the order of execution. The execution order of each process should be determined by its function and internal logic, and should not constitute any limitation on the implementation process of the embodiments of the present invention.

The flowcharts and block diagrams in the drawings of the present invention illustrate the possible system architectures, functions, and operations of the system, method, and computer program product according to various embodiments of the present application. In this regard, each block in the flowchart or block diagram may represent a module, program segment, or part of the code, and the module, program segment, or part of the code includes one or more executable instructions for realizing the specified logical function. It should also be noted that in some alternative implementations, the functions marked in the blocks may also occur in a different order than that marked in the drawings. For example, two blocks shown in succession can actually be executed substantially in parallel, or they can sometimes be executed in the reverse order, depending on the functions involved. It should be particularly noted that each block in the block diagram and/or flowchart, and the combination of blocks in the block diagram and/or flowchart, can be implemented by a dedicated hardware-based system that performs the specified functions or operations, or by a combination of dedicated hardware and computer instructions.

The above is a detailed introduction to a data cable and massage control method thereof disclosed in the embodiments of the present invention. Specific examples are used in this article to explain the principle and implementation of the present invention. The description of the above embodiments is only used to help understand the method and core idea of the present invention; at the same time, for those of ordinary skill in the art, according to the idea of the present invention, there will be changes in the specific implementation and application scope. In summary, the content of this specification should not be understood as a limitation to the present invention. Any modifications, equivalent replacements, and improvements made within the principles of the present invention should be included in the protection scope of the present invention.

## Claims

1. A data cable (1), wherein the data cable (1) comprises a first input/output module (11), a first transmission line (210), a massage module (13), a second transmission line (211), and a second input/output module (14) which are electrically connected in sequence, and a main control module (12) electrically connected to the massage module (13);
the first input/output module (11) and the second input/output module (14) are any one of USB Type-A connector, USB Type-B connector, USB Type-C connector or Lightning connector; the first input/output module (11) is connected to an external device and the second input/output module (14) is connected to another external device for the transmission of electrical or data signals between the two external devices, and simultaneously transmits the electrical signals to the main control module (12) and the massage module (13);
the main control module (12) sends out control signals for controlling the start-stop or adjustment modes of the massage module (13);
the massage module (13) comprises a housing (133) which is bendable, a flexible circuit structure (132) and massage functional components (131) which are disposed within the housing (133), the data cable (1) comprises a main charging circuit (21) for charging and a massage branch circuit (22) electrically connected to the main charging circuit (21), the main charging circuit (21) comprises a first transmission line (210), the flexible circuit structure (132), and a second transmission line (211) which are electrically connected in sequence;
the massage functional components (131) are arranged on the massage branch circuit (22), positioned along the length direction of the flexible circuit structure (132), and perform massage actions based on the control signals.

2. The data cable (1) as claimed in claim 1, wherein: the massage module (13) is in the shape of an overall flattened elongated strip; the massage module (13) has a width in the range of 15mm~40mm, a length in the range of 100mm~300mm, and a thickness in the range of 3mm~20mm.

3. The data cable (1) as claimed in claim 1, wherein: the number of the massage functional components (131) is as at least two, and the massage functional components (131) are arranged at intervals in sequence along the length direction of the massage module (13) on the central axis of the massage module (13); the main control module (12) is also provided with a display device (121), the display device (121) is an indicator light or a display screen connected to the main control module (12), which is used for displaying the working status of the massage functional components (131).

4. The data cable (1) as claimed in claim 1, wherein: the data cable (1) comprises a bidirectional signal recognition circuit for detecting the connection of the external devices and recognizing the direction of transmission of electrical and data signals, as long as either the first input/output module (11) or the second input/output module (14) is connected to the external device, the external device can supply power to the main control module (12) and the massage module (13); the bidirectional signal recognition circuit is implemented by USB-OTG technology; the data cable (1) further comprises a first circuit board (23) and a second circuit board (24), the first transmission line (210) is electrically connected to the flexible circuit structure (132) via the first circuit board (23), the second transmission line (211) is electrically connected to the flexible circuit structure (132) via the second circuit board (24).

5. The data cable (1) as claimed in claim 4, wherein the massage branch circuit (22) further comprises a voltage reduction unit (220) and a voltage boosting unit (221), which are electrically connected, a first voltage introduced from the first input/output module (11) or a second voltage introduced from the second input/output module (14) is converted into a third voltage via the voltage reduction unit (220), the third voltage is converted into a fourth voltage via the voltage boosting unit (221); the third voltage or the fourth voltage is introduced into the massage functional components (131); the voltage reduction unit (220) and the voltage boosting unit (221) are integrated on the first circuit board (23) or the second circuit board (24); or the main control module (12) is integrated on the first circuit board (23) or the second circuit board (24), or the main control module (12) is integrated on a third circuit board independent of the first circuit board (23) and the second circuit board (24).

6. The data cable (1) as claimed in claim 4, wherein: the main charging circuit (21) comprises at least a positive power transmission line, a ground transmission line, a positive data transmission line, a negative data transmission line, and a configuration channel line; the massage branch circuit (22) leads out at least two transmission pins (222) electrically connected to the massage functional components (131); the pads of the transmission pins (222) are arranged on the flexible circuit structure (132), the first circuit board (23) or the second circuit board (24).

7. The data cable (1) as claimed in claim 6, wherein: the massage functional components (131) are massage electrode sheets (26), and each massage electrode sheet (26) comprises a main body (260), as well as conductive pins (262) and a conductive working surface (261) arranged on the main body (260); the transmission pins (222) comprise an EMS positive pin and an EMS negative pin; the conductive pins (262) of the at least one massage electrode sheet 26 are electrically connected to the EMS positive pin, and the conductive pins (262) of the at least another one massage electrode sheet 26 are electrically connected to the EMS negative pin; or the massage functional components (131) are vibration motors (27), each vibration motor (27) comprises a motor positive pin and a motor ground pin, the transmission pins (222) comprise a transmission positive pin and a transmission ground pin, the motor positive pins and the motor ground pins are electrically connected to the transmission positive pin and transmission ground pin respectively.

8. The data cable (1) as claimed in claim 1, wherein: the housing (133) is provided with flexible massage parts (30) corresponding to the massage functional components (131), massage structures (300) are arranged on opposite sides of each massage part (30), and an elastic structure (31) is arranged on a peripheral side of the massage structure (300) of at least one side of each massage part (30); each elastic structure (31) comprises a first annular groove (310), an annular protrusion (311), and a second annular groove (312) which are connected in sequence; each massage part (30) is provided with a cavity (301) accommodating corresponding massage functional component (131), and a boss (302) is provided on the inner wall of each cavity (301).

9. The data cable (1) as claimed in claim 7, wherein: the housing (133) is provided with a matching receiving groove (42) corresponding to the flexible circuit structure (132); each massage electrode sheet (26) is mounted on the housing (133) via a support member (40); each support member (40) is provided with a hollow cavity (400) for receiving the main body (260) of corresponding massage electrode sheet (26), the conductive working surface (261) of each massage electrode sheet (26) is exposed out of the hollow cavity (400) of corresponding support member (40), a transverse groove (401) communicating with the hollow cavity (400) of each support member (40) is provided on an end of each support member (40) away from the conductive working surface (261) of corresponding massage electrode sheet (26), and the conductive pins (262) of each massage electrode sheet (26) are exposed out of the transverse groove (401) of corresponding support member (40); an annular groove (402) is provided on the peripheral side of each support member (40), and the housing (133) is provided with openings (41), and each annular groove (402) is clamped with the edge of each opening (41).

10. The data cable (1) as claimed in claim 1, wherein: the main control module (12) comprises a function control unit (122), the function control unit (122) being electrically connected to the massage module (13) for receiving user instructions and outputting corresponding control signals to the massage module (13) in accordance with the user instructions; the function control unit (122) comprises a signal input unit (123), the user instructions being received via the signal input unit (123); or the user instructions being input via the external device connected to the first input/output module (11) or the external device connected to the second input/output module (14), and an application program for receiving the user instructions may be provided on the external device; after the user inputting the user instructions by the application program, the user instructions are transmitted to the main control module (12) via the first input/output module (11) or the second input/output module (14) to control the start-stop or adjustment modes of the massage module (13).

11. The data cable (1) as claimed in claim 1, wherein: the massage actions comprise any one type of, or a combination of any several types of, a vibration signal, an electrical pulse signal, and a heat signal; and the adjustment modes comprise any one type of, or a combination of any several types of, a massage intensity adjustment, a massage mode adjustment, and a timing adjustment.

12. The data cable (1) as claimed in claim 1, wherein: the data cable (1) further comprises a massage accessory (50) which is detachable, the main control module (12) is provided with an output interface (51), the massage accessory (50) is electrically connected to the main control module (12) via the output interface (51); the massage accessory (50) comprises any one type of, or a combination of any several types of, a vibration massage member, a heating massage member, an electric pulse massage member.

13. The data cable (1) as claimed in claim 1, wherein the data cable (1) is further provided with a buckle assembly (60), the buckle assembly (60) comprises a buckle member (61) and a cable clamp member (62) which are detachably connected, the cable clamp member (62) being provided with a first through-hole (620), a second through-hole (621) and a locking and unlocking device (622); both ends of the data cable (1) pass through the first through-hole (620) and the second through-hole (621) respectively and are fixed or movable with respect to the cable clamp member(62) by the locking and unlocking device (622); or the cable clamp member(62) comprises a male part (627) and a female part (628) which are detachably connected, the first through-hole (620) and the second through-hole (621) are provided in the male part(627) and the female part(628) respectively, and at least one of the male part(627) and the female part(628) is provided with the locking and unlocking device (622).

14. A massage control method for a data cable (1), the data cable (1) comprises a first input/output module (11), a massage module (13) and a second input/output module (14) connected in sequence, and a main control module (12) electrically connected to the massage module (13); the first input/output module (11) is connected to an external device, the second input/output module (14) being connected to another external device, realizing the transmission of electrical or data signals between the two external devices, and simultaneously transmitting electrical or data signals to the main control module (12) and the massage module (13); the method comprises:obtaining control signals from the main control module (12) based on user instructions; inputting electrical signals from the first input/output module (11) or the second input/output module (14) to supply power to the massage module (13); based on the control signals, controlling the massage module (13) to perform massage actions.

15. The massage control method for a data cable (1) as claimed in claim 14, wherein the method further comprises:
controlling different working modes of the data cable (1) based on user instructions;
the working modes comprise the data cable (1) transmitting only data signals and/or electrical signals, the data cable (1) transmitting data signals and/or electrical signals while performing massage actions, and the data cable (1) performing only massage actions.
